# FASCICULE DE BREVET EUROPEEN

(11) **EP 4 456 782 B1**
(45) Date de publication et mention de la délivrance du brevet: **03.06.2026**
(21) Numéro de dépôt: 22839767.5
(22) Date de dépôt: 16.12.2022
(51) Int. Cl.: A61B 5/00, A61N 1/02, A61N 1/36, A61B 5/0533, A61B 5/0535, A61B 5/0537, G01G 19/50

(54) **STATION DE MESURE AVEC MESURE DE L'ACTIVITÉ SUDORALE**
MESSSTATION MIT SCHWEISSMESSUNG
MEASUREMENT STATION WITH MEASUREMENT OF PERSPIRATION

(30) Priorité: 31.12.2021 FR 2114739
(43) Date de publication de la demande: 06.11.2024
(73) Titulaire: Withings, 92130 Issy-Les-Moulineaux (FR)
(72) Inventeur: KRAUSE, Thomas, 92130 Issy-les-Moulineaux (FR); JOUSSAIN, Antoine, 92130 Issy-les-Moulineaux (FR); WANG, Jean-Louis, 92130 Issy-les-Moulineaux (FR); BREDIN, Jérôme, 92130 Issy-les-Moulineaux (FR); YU, Roger, 92130 Issy-les-Moulineaux (FR); BRAC DE LA PERRIÈRE, Brice, 92130 Issy-les-Moulineaux (FR); CALLENS, Victorine, 92130 Issy-les-Moulineaux (FR)
(74) Mandataire: Withings IP
(86) Numéro de dépôt international: PCT/EP2022/086492
(87) Numéro de publication internationale: WO 2023/126220

(56) Documents cités:
- WO-A1-2014/033120
- WO-A1-2020/081472
- WO-A1-2021/164561
- CN-A- 112 022 102
- US-A1- 2001 049 479
- US-A1- 2015 362 360
- US-A1- 2017 065 185
- US-A1- 2018 235 508
- US-A1- 2019 199 824

## Description

### Domaine technique

. La présente description concerne la surveillance de la santé d'un utilisateur et plus spécifiquement concerne les stations de mesure permettant de mettre en œuvre une ou plusieurs mesures de signaux biométriques (ou paramètres physiologiques) d'un utilisateur.

. Au moins une des données suivantes est déterminée par la station de mesure de la présente demande : poids ou masse, électrocardiogramme (ECG), impédancemétrie (analyse d'impédance du corps humain), dont impédance-pléthysmogramme (IPG) impedance-cardiogramme (ICG) et bioimpédance (« *bioimpedance analysis* » BIA, pour la masse de graisse, d'eau, de muscles, etc.), photoplethysmogramme (PPG), ballistocardiogramme (BCG), analyse de la conductance électrochimique de la peau (« *ESC analysis* » pour « *electrochemical skin conductance* » ou plus simplement « ESC » dans la présente description) et évaluation de la fonction sudorale (parfois appelée « *sudogramme* » dans la présente domande), rythme cardiaque (« *heart rate* », HR), vitesse d'onde de pouls vitesse d'onde de pouls (« *pulse wave velocity* », PWV), etc.

### Etat de la technique

. Le document WO2010/122252, de 2010, décrit une balance connectée avec mesure de poids et de bioimpédance. Les documents EP3087914 et EP3095380, de 2015, décrivent quant à eux une balance connectée permettant d'obtenir des informations sur l'état cardiovasculaire de l'utilisateur, avec notamment une mesure de PTT (« *pulse transit time* ») à l'aide d'un BCG et d'un IPG. Le document WO2021/164561 décrit une balance avec poignée permettant de mesurer un poids, d'effectuer un ECG multi-voies et d'effectuer une BIA segmentale. Le document WO 2020/081472 décrit une station de mesure de type balance comprenant des électrodes plantaires disposées en groupes gauche et droit, permettant notamment la réalisation de mesures d'impédancemétrie corporelle, telles que l'IPG par injection de courant alternatif.

. Le développement de nouveaux dispositifs de mesure pouvant être utilisé au domicile est souhaitable.

### Présentation de l'invention

. La présente description vise à proposer une station de mesure permettant l'obtention de différentes mesures de signaux biométriques, avec une qualité accrue.

. L'invention est définie dans les revendications.

. Dans un mode de réalisation, la description présente une station de mesure comprenant :
- un groupe gauche d'électrodes, comprenant au moins deux électrodes électriquement indépendantes agencées pour être au contact du dessous d'un pied gauche d'un utilisateur,
- un groupe droite d'électrodes, comprenant au moins deux électrodes électriquement indépendantes agencées pour être au contact du dessous d'un pied droit de l'utilisateur,
- une source de tension continue,
- un commutateur configuré pour activer et désactiver une configuration dite à courant continu, dite configuration CC, dans laquelle au moins une électrode, dite électrode active, d'au moins un groupe d'électrodes est connectée à la source de tension continue.

. En particulier, une électrode de chaque groupe est connectée à la source de tension continue (à ses deux bornes opposées). Un groupe fonctionne en cathode et l'autre groupe fonctionne en anode.

. Dans un mode de réalisation, en configuration CC, au moins une électrode, dite électrode passive, d'un groupe d'électrode n'est pas reliée à la source de tension continue. L'électrode passive peut être une électrode connectée en haute impédance, par exemple supérieure à 500 kOhm. Dans une variante, au moins une électrode de chaque groupe d'électrodes n'est pas reliée à la source de tension continue.

. Dans un mode de réalisation, la station de mesure comprend en outre une source de courant alternatif et le commutateur est configuré pour sélectivement activer ou désactiver une configuration dite à courant alternatif, dite configuration AC (ACs, ACf, ACb), dans laquelle au moins une électrode parmi les électrodes groupe gauche d'électrodes et du groupe droite d'électrodes est connectée à la source de courant alternatif. Le commutateur est configuré pour commuter au moins entre la configuration CC et la configuration AC. Les configurations CC et AC ne peuvent être activées simultanément. La configuration AC permet de faire une BIA (entre les jambes, et segmentale avec une poignée), un IPG entre les jambes et un IPG dans le pied.

. Dans un mode de réalisation, dans une configuration AC (ACf, ACb), le commutateur est configuré pour connecter au moins deux électrodes parmi les électrodes du groupe gauche d'électrodes et du groupe droit d'électrodes aux bornes de la source de courant alternatif, Cet agencement permet de faire du BIA entre les jambes, de l'IPG entre les jambes et de l'IPG dans le pied. Le commutateur est en outre configuré pour connecter au moins deux électrodes parmi le groupe gauche d'électrodes et le groupe droite d'électrodes aux bornes d'un voltmètre.

. Dans un mode de réalisation, dans une configuration AC (ACb), le commutateur est configuré pour connecter au moins une électrode du groupe gauche et au moins une électrode du groupe droite aux bornes de la source de courant alternatif. Cet agencement permet de faire du BIA entre les jambes et de l'IPG entre les jambes. Le commutateur est en outre configuré pour connecter au moins une électrode du groupe gauche et au moins une électrode du groupe droite aux bornes du voltmètre.

. Dans un mode de réalisation, en configuration AC (ACf), le commutateur est configuré pour connecter au moins une électrode d'un groupe à une borne de la source de courant et une autre électrode du même groupe à une autre borne de la source de courant. Cet agencement permet de faire de l'IPG dans le pied. Le commutateur est en outre configuré pour connecter au moins deux électrodes d'un même groupe aux bornes d'un voltmètre. En particulier, les deux électrodes connectées au voltmètre ne sont pas adjacentes.

. Dans un mode de réalisation, le groupe gauche (LG) et/ou le groupe droite (RG) comprend chacun trois électrodes électriquement indépendantes, et, en configuration CC, le commutateur est configuré pour connecter au moins deux électrodes du groupe gauche (LG) et/ou au moins deux électrodes du groupe droite (RG) à la source de tension continue.

. Dans un mode de réalisation, le groupe gauche (LG) et/ou le groupe droite (RG) comprend chacun quatre électrodes électriquement indépendantes, et, en configuration CC, le commutateur est configuré pour connecter au moins trois électrodes du groupe gauche et/ou au moins trois électrodes du groupe droite à la source de tension continue.

. Dans un mode de réalisation, le groupe gauche (LG) et/ou le groupe droite (RG) comprend chacun cinq électrodes électriquement indépendantes, et, en configuration CC, le commutateur est configuré pour relier au moins quatre électrodes du groupe gauche (LG) et/ou au moins quatre électrodes du groupe droite (RG) à la source de tension continue.

. Dans un mode de réalisation, le groupe gauche (LG) et/ou le groupe droite (RD) comprend chacun au moins six électrodes électriquement indépendantes, et, en configuration CC, le commutateur est configuré pour relier au moins cinq électrodes du groupe gauche (LG) et/ou au moins cinq électrodes du groupe droite (RD) à la source de tension continue. En configuration AC, au moins deux électrodes inactives peuvent se trouver entre les deux électrodes connectées au voltmètre. Une de ces électrodes inactives en configuration AC peut être l'électrode passive en configuration CC.

. Dans un mode de réalisation, le groupe gauche (LG) et/ou le groupe droite (RG) comprend chacun au moins sept électrodes électriquement indépendantes, et, en configuration CC, le commutateur est configuré pour relier au moins six électrodes du groupe gauche (LG) et/ou au moins six électrodes du groupe droite (RG) à la source de tension continue. En configuration AC au moins trois électrodes inactives peuvent se trouver entre les deux électrodes connectées au voltmètre, une de ces électrodes inactives peut est l'électrode passive en configuration CC.

. Dans un mode de réalisation, en configuration CC, l'électrode passive est située entre deux électrodes actives.

. Dans un mode de réalisation, les électrodes sont arrangées côte à côte, espacées les unes des autres, dans le sens d'une longueur de la station de mesure.

. Dans un mode de réalisation, les électrodes sont sous forme de bandes parallèles entre elles.

. Dans un mode de réalisation, les électrodes sont espacées les unes des autres, dans le sens d'une longueur de la station de mesure. Cela permet de limiter les effets du placement avant/arrière du pied. Dans un mode de réalisation, les électrodes extrémales le long de la longueur sont plus larges, afin de limiter les effets de placement de pieds dans le sens de la longueur.

. Dans un mode de réalisation, les électrodes configurées pour être actives en configuration CC ont une dimension dans le sens d'une longueur de la station de mesure qui est égal ou supérieure à celle des électrodes configurées pour être passives en configuration CC.

. Dans un mode de réalisation, pour un groupe droite ou gauche d'électrodes, les électrodes configurées pour être actives en configuration CC couvrent en surface (Sa) au moins 50% de la surface (Se) de l'enveloppe convexe définie par toutes les électrodes du groupe. Alternativement ou complémentairement, la distance (Dmax) entre les électrodes extrémales, électrodes incluses, le long d'une longueur de la station de mesure est d'au moins 20 cm. Alternativement ou complémentairement, les électrodes s'étendent sur une largeur de la station sur au moins 10 cm, voire au moins 15 cm. Cela permet de limiter les effets du placement latéral du pied.

. Dans un mode de réalisation, les électrodes du groupe gauche s'étendent le long d'une largeur de la station de mesure sur plus de 40% de la largeur de la station de mesure et les électrodes du groupe droit s'étendent le long d'une largeur de la station de mesure sur plus de 40% de la largeur de la station de mesure.

. Dans un mode de réalisation, les électrodes comprennent un matériau en oxyde d'indium-étain, ITO.

. Dans un mode de réalisation, la source de tension continue est configurée pour sélectivement appliquer à une paire d'électrodes, dites actives, des paliers successifs de tension constante, lesdites électrodes de la paire constituant une anode et une cathode. Les valeurs de tension des paliers successifs peuvent être décroissantes (c'est-à-dire que chaque palier présente une valeur de tension inférieure à celle du palier précédent) et/ou durent entre 500ms et 2s chacun.

. Dans un mode de réalisation, la station de mesure selon l'une quelconque des revendications précédentes, comprenant en outre un capteur de poids.

. Dans un mode de réalisation, la station de mesure comprend une base comprenant le groupe gauche d'électrode et le groupe droite d'électrode, et comprend une poignée avec au moins une électrode apte à être au contact de la main. En configuration CC, le commutateur est configuré pour connecter l'électrode de la poignée à une haute impédance. En particulier, le commutateur peut être configuré pour connecter toutes les électrodes de la base aux bornes de la source de tension continue.

. Dans un mode de réalisation, les électrodes du groupe gauche et droite comprennent un matériau en oxyde d'indium-étain, ITO.

. La description présente aussi une station de mesure comprenant :
au moins une paire d'électrodes comprenant un matériau en oxyde d'indium-étain, ITO,
une source de tension continue configurée pour sélectivement appliquer à la paire d'électrodes, dites actives, des paliers de tension continue, lesdites électrodes de la paire constituant une anode et une cathode. Cette station de mesure peut effectuer un ESC.

. La description présente aussi dans un mode de réalisation, une station de mesure comprenant :
- une base apte à recevoir au moins un pied d'un utilisateur,
- un poignée apte à recevoir au moins une main d'un utilisateur,
- un câble, reliant la base à la poignée,
dans laquelle la poignée comprend un interrupteur-maitre de poignée configuré pour alterner entre une position d'électrocardiogramme et une position d'impédancemétrie et la base comprend un interrupteur-maitre configuré pour alterner entre une position d'électrocardiogramme et une position d'impédancemétrie.

. Dans un mode de réalisation, la poignée comprend quatre électrodes, chaque électrode étant connectée à un interrupteur pour connecter ladite électrode à un circuit électrique d'électrocardiogramme ou à un circuit d'impédancemétrie, le circuit électrique ECG et le circuit d'impédancemétrie étant reliés à l'interrupteur-maitre. Les quatre électrodes permettent à la poignée d'effectuer une mesure de BIA (notamment segmentale) et un ECG.

. Dans un mode de réalisation, le circuit électrique d'électrocardiogramme et le circuit électrique d'impédancemétrie permettent de traiter les signaux électriques avant qu'ils ne transitent par le câble.

. La description présente aussi un procédé de mesure (par exemple d'acquisition d'ESC) à l'aide d'une station telle que décrite précédemment, le procédé comprenant une étape de commutation, à l'aide du commutateur, entre deux configurations différentes.

### Description des figures

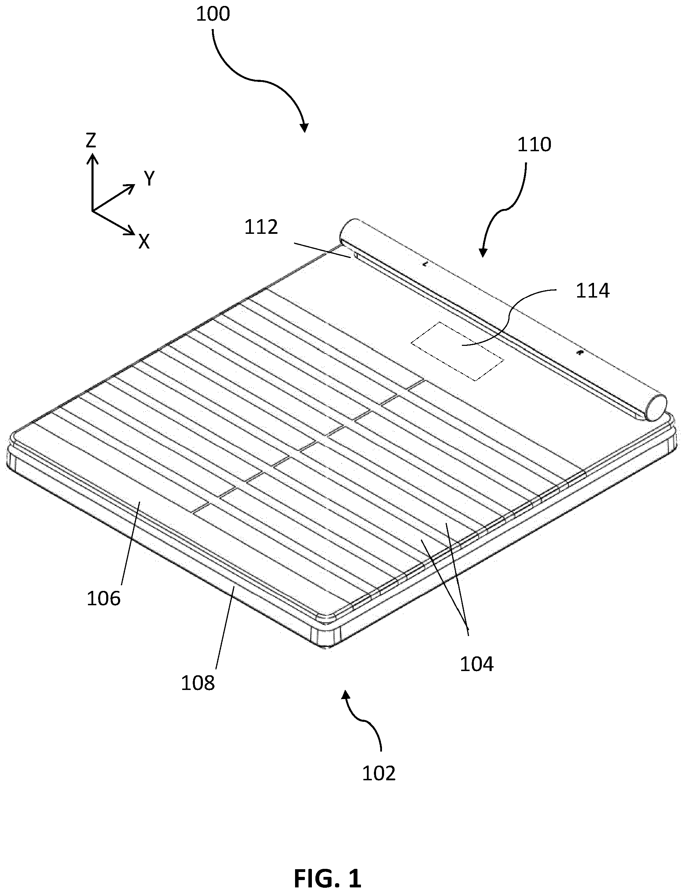

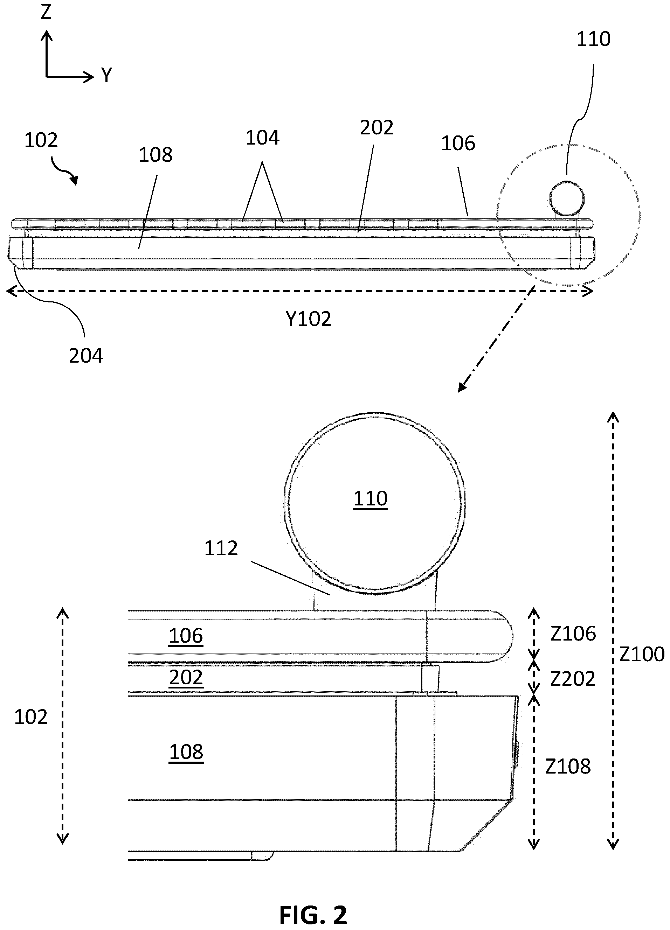

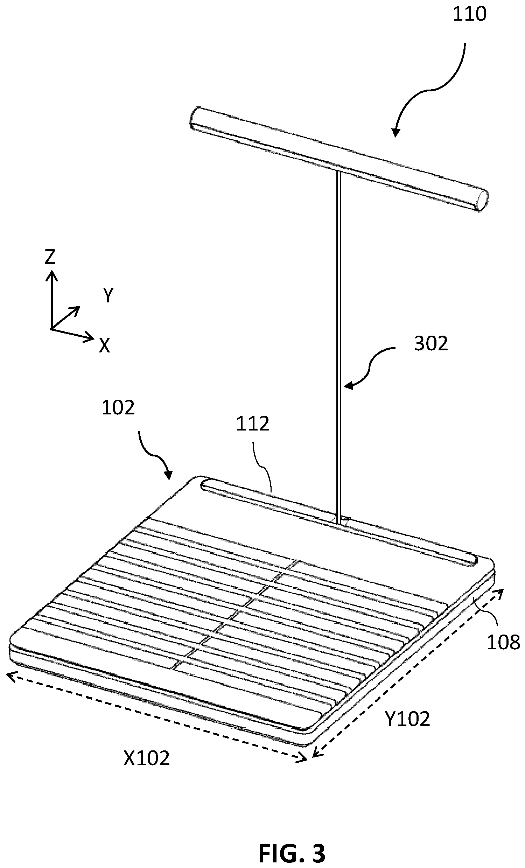

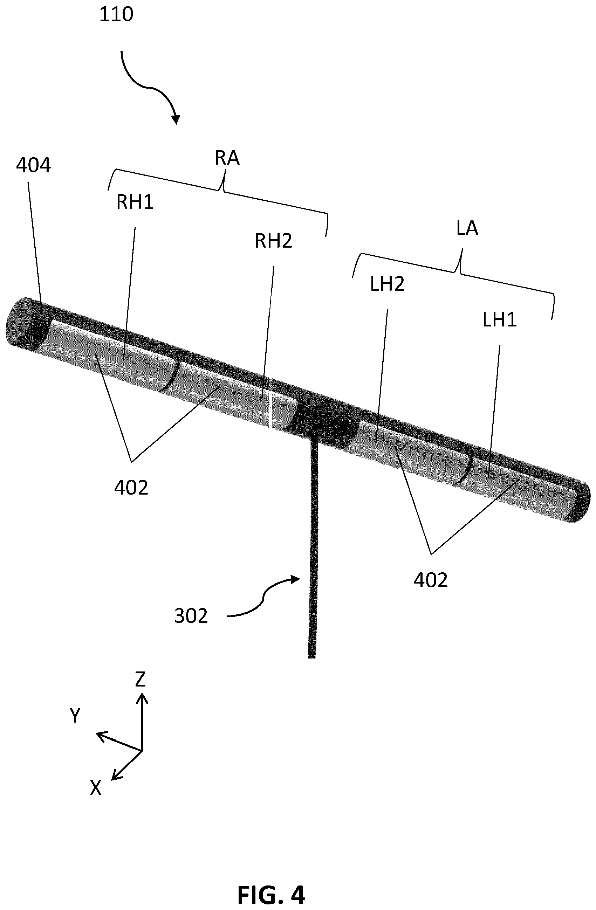

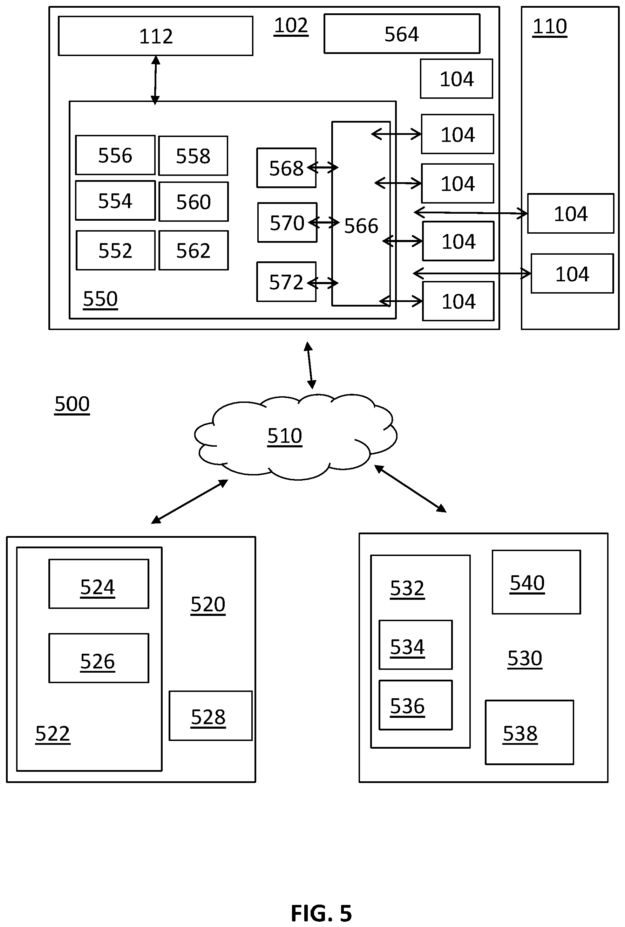

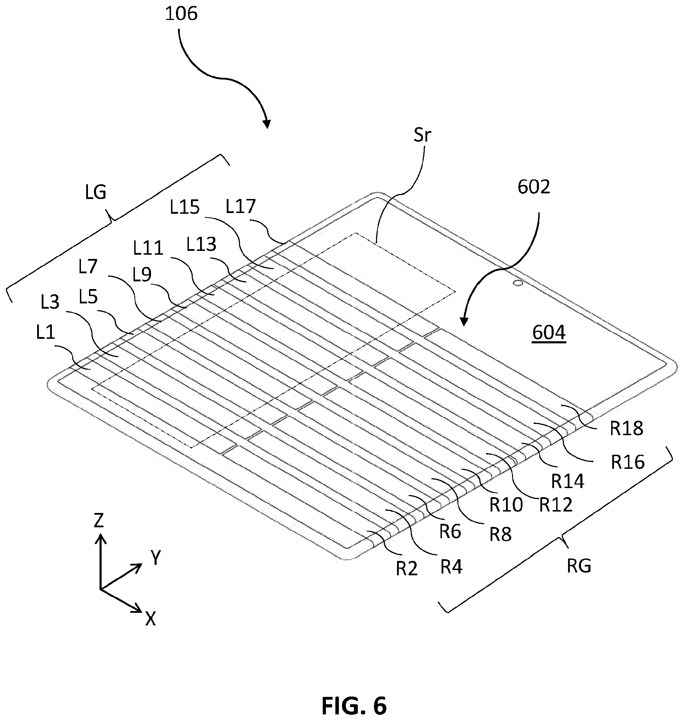

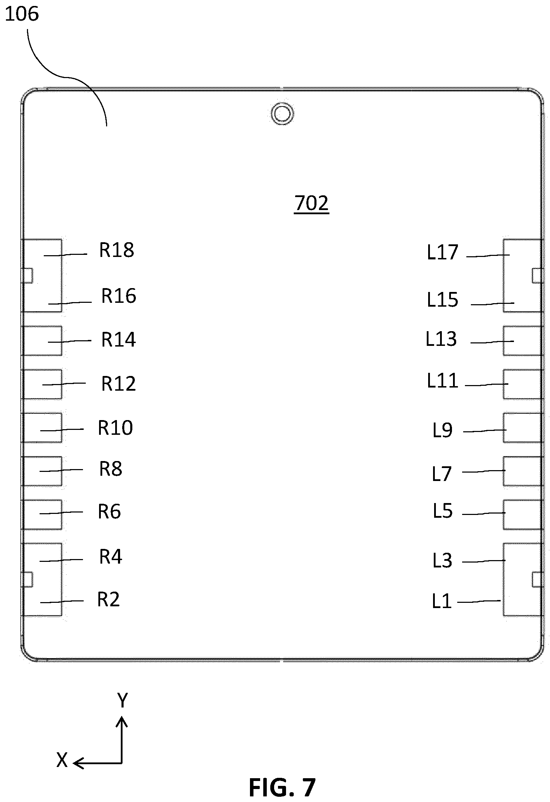

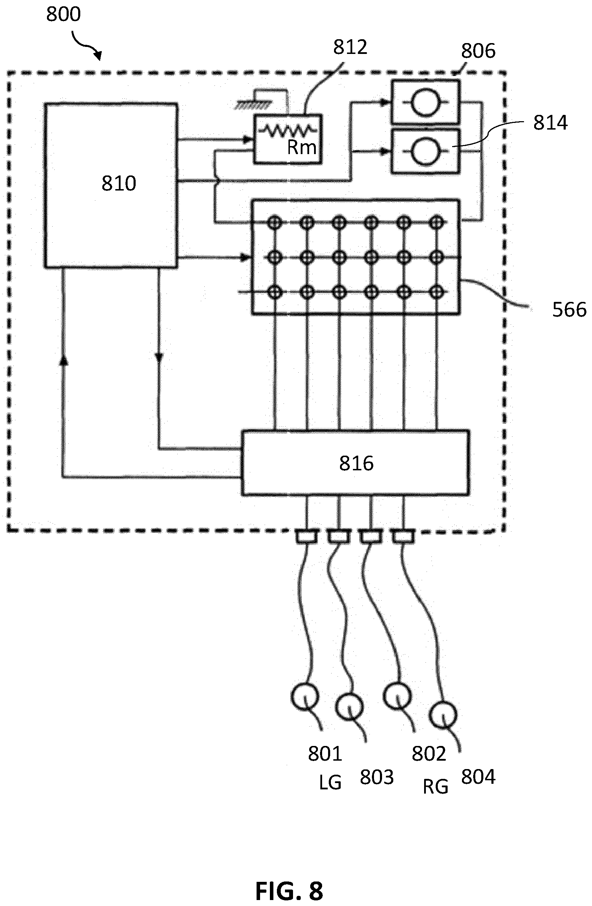

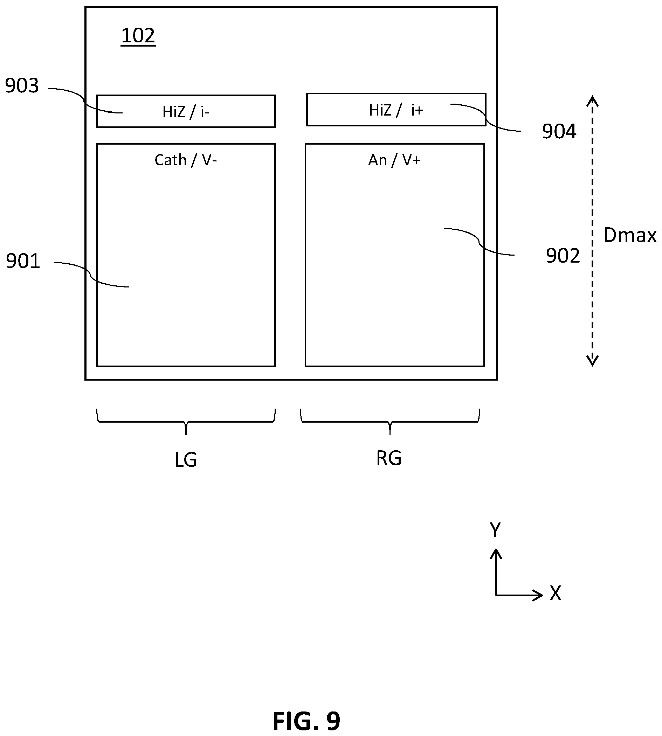

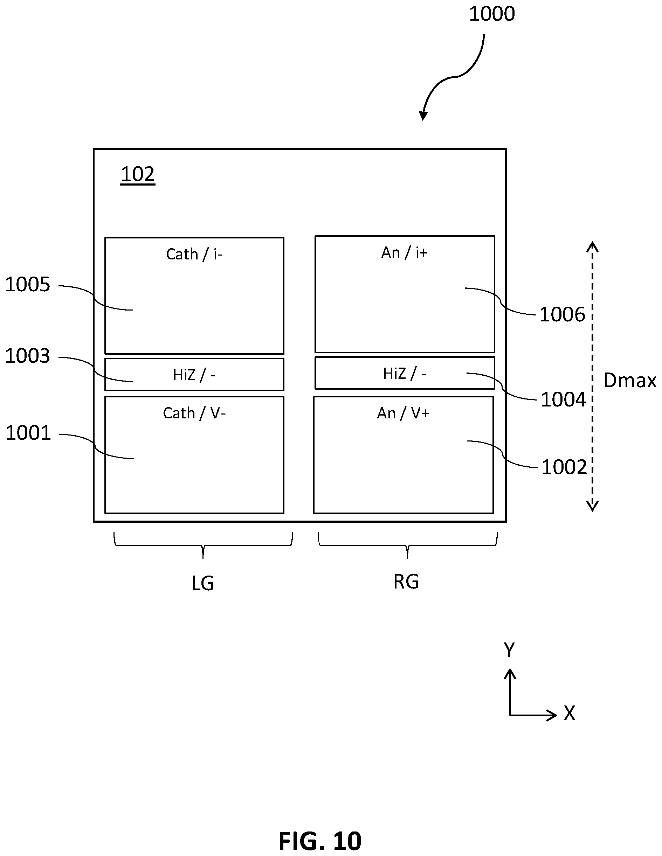

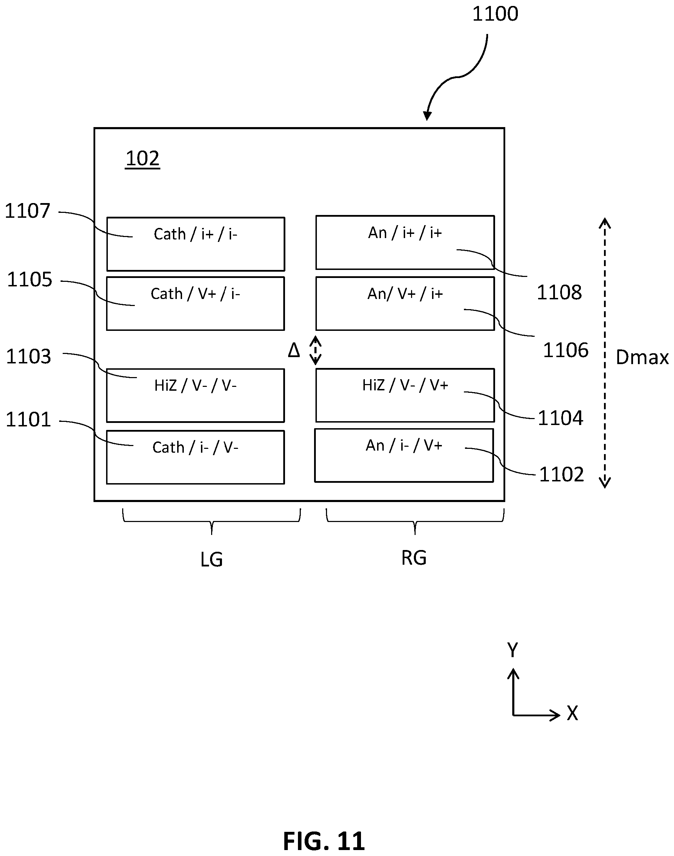

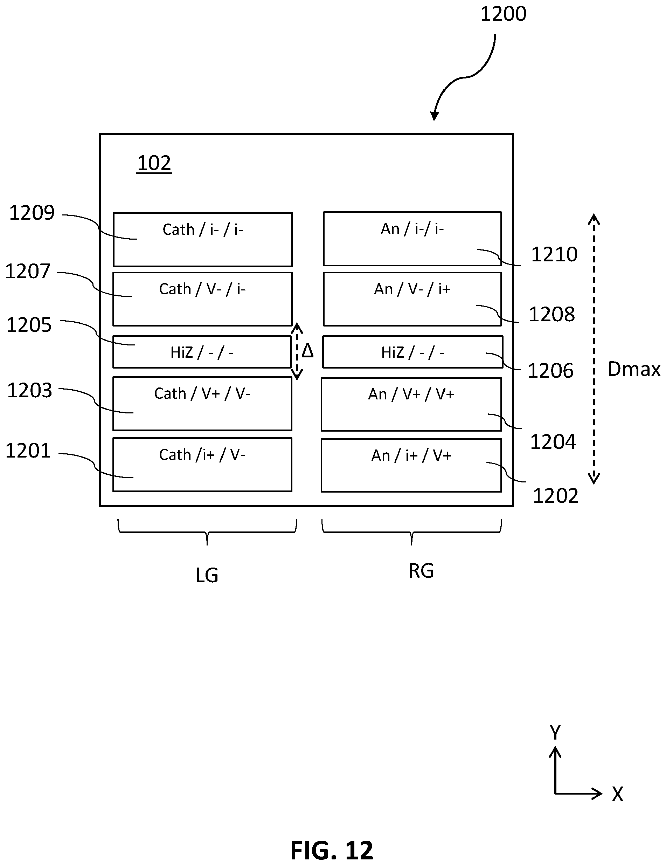

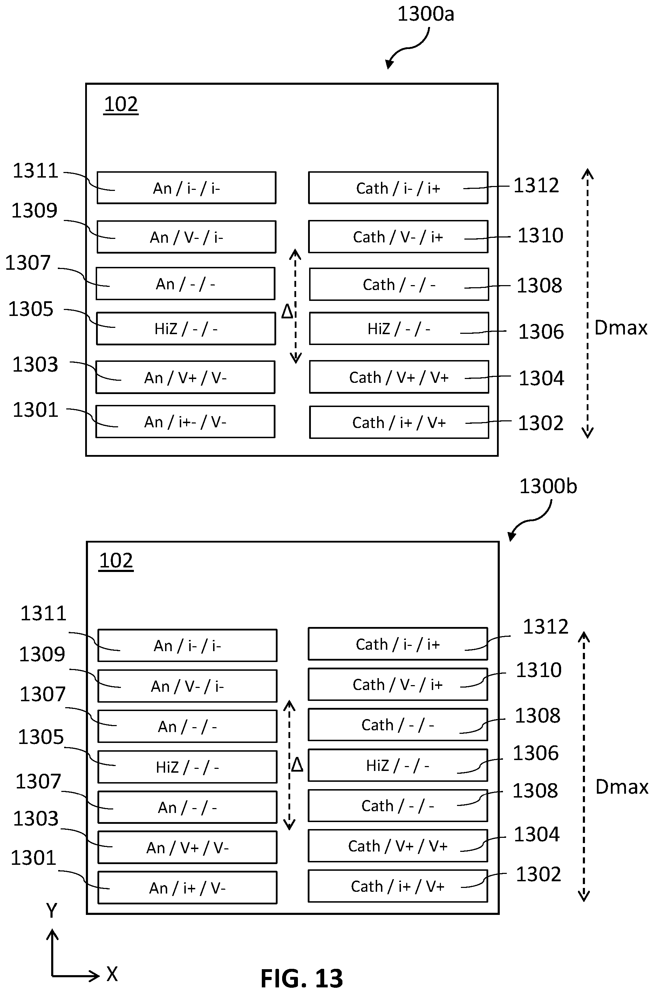

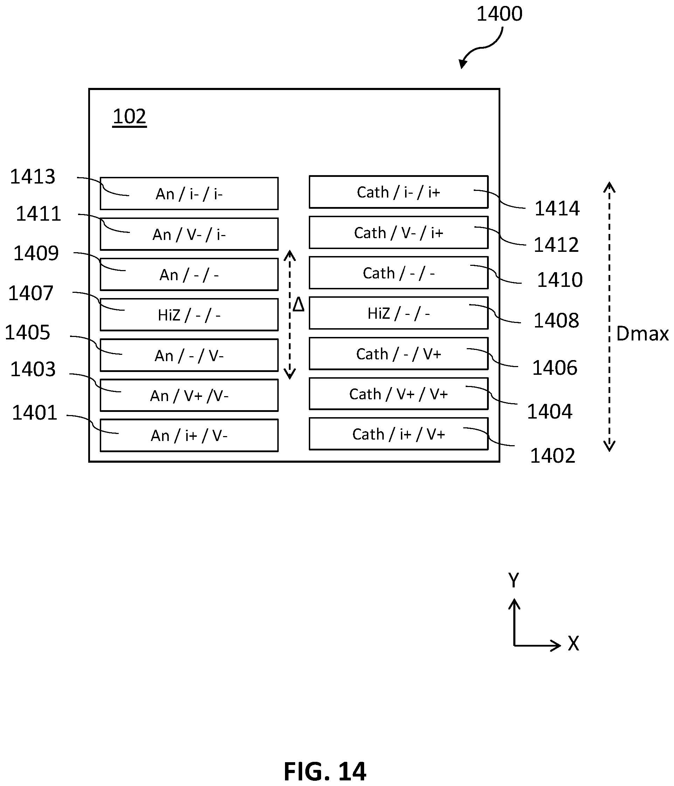

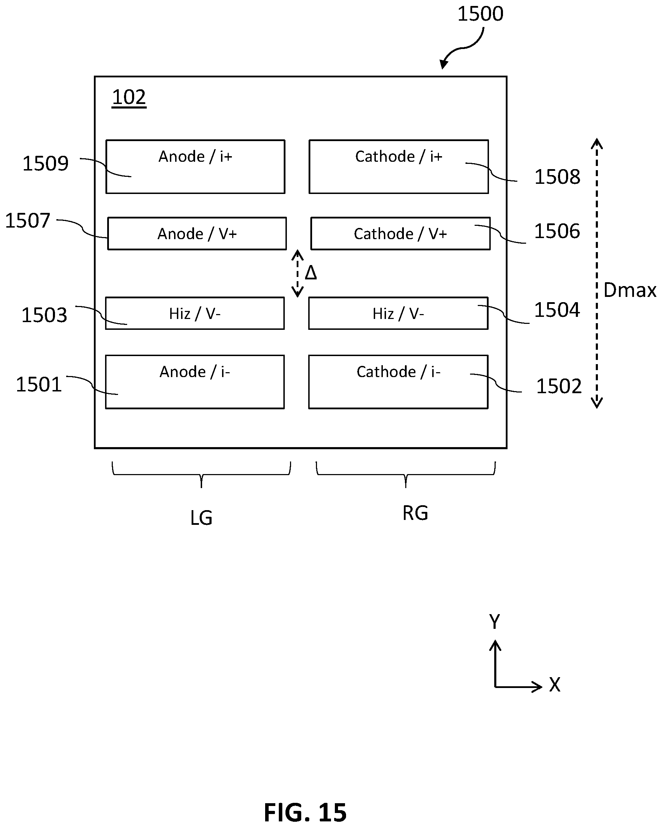

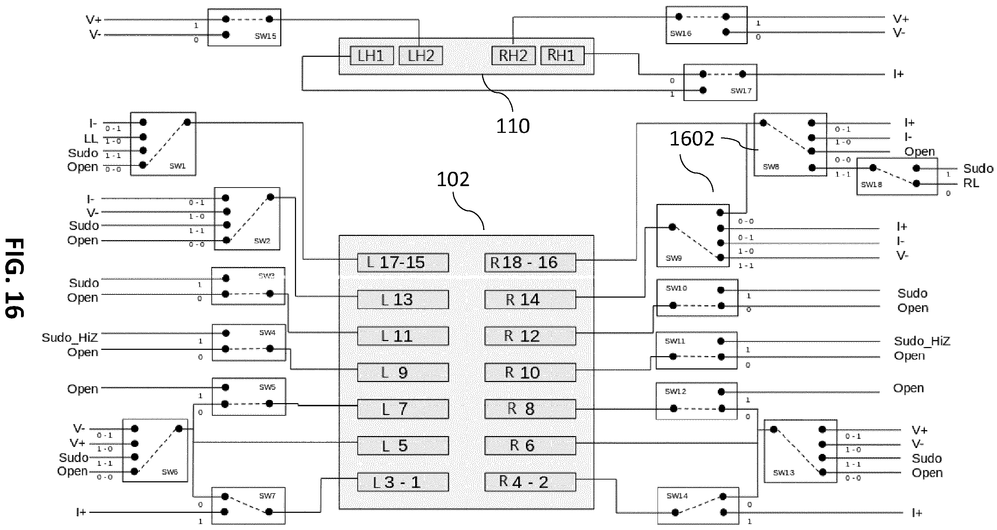

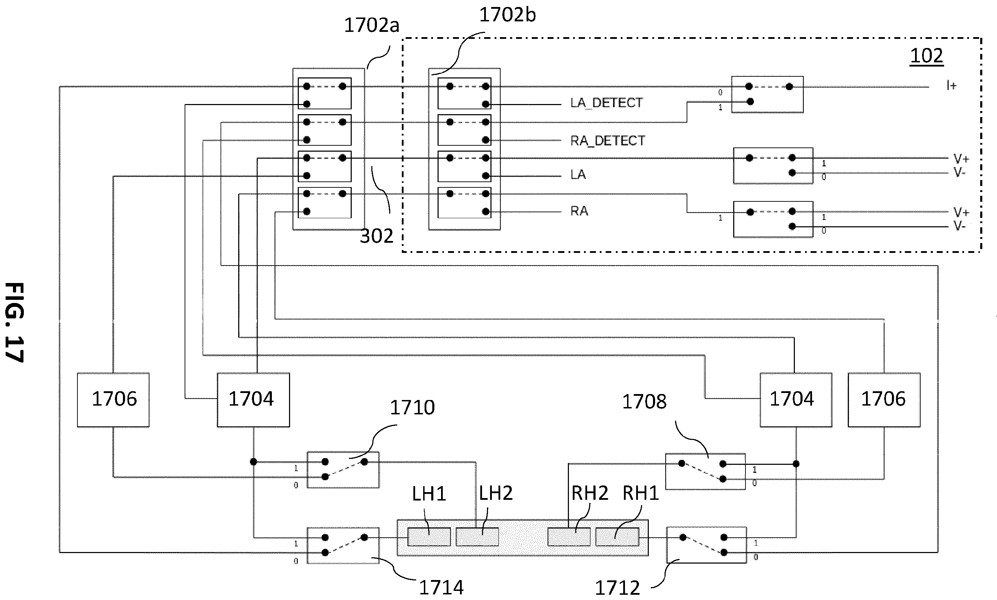

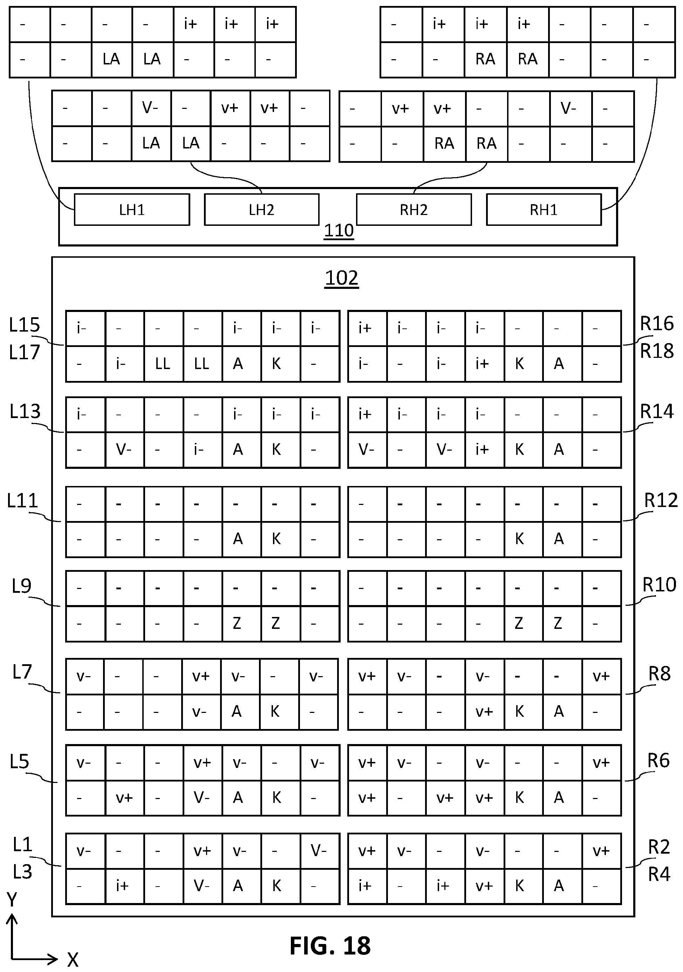

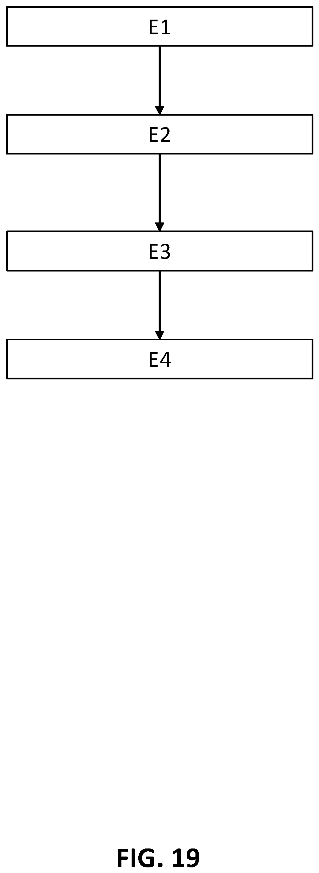

. Les figures suivantes illustrent les éléments décrits dans la présente description.
. FIG. 1 : la figure 1 représente une vue tridimensionnelle d'une station de mesure avec une poignée, selon un mode de réalisation ;
. FIG. 2 : la figure 2 représente une vue latérale de la station de la figure 1 ;
. FIG. 3 : la figure 3 représente une vue tridimensionnelle de la station de la figure 1, mais avec la poignée en position déployée ;
. FIG. 4 : la figure 4 représente une vue détaillée de la poignée ;
. FIG. 5 : la figure 5 représente une vue schématique de la station de mesure et de son environnement ;
. FIG. 6 : la figure 6 illustre une vue tridimensionnelle d'une plaque de mesure isolée ;
. FIG. 7 : la figure 7 illustre une vue du dessous la plaque de mesure de la figure 6 ;
. FIG 8 : la figure 8 représente une vue schématique des composants de la station de mesure pour notamment effectuer un ESC ;
. FIG. 9 : la figure 9 représente un agencement d'électrodes avec deux électrodes indépendantes par groupe gauche ou droite d'électrodes ;
. FIG. 10 : la figure 10 représente un agencement d'électrodes avec trois électrodes indépendantes par groupe gauche ou droite d'électrodes ;
. FIG. 11 : la figure 11 représente un agencement d'électrodes avec quatre électrodes indépendantes par groupe gauche ou droite d'électrodes ;
. FIG. 12 : la figure 12 représente un agencement d'électrodes avec cinq électrodes indépendantes par groupe gauche ou droite d'électrodes ;
. FIG. 13 : la figure 13 représente un agencement d'électrodes avec six électrodes indépendantes par groupe gauche ou droite d'électrodes ;
. FIG. 14 : la figure 14 représente un agencement d'électrodes avec sept électrodes indépendantes par groupe gauche ou droite d'électrodes ;
. FIG. 15 : la figure 15 représente un agencement d'électrodes avec des électrodes extrémales plus importantes ;
. FIG. 16 : la figure 16 illustre un schéma illustrant une partie du commutateur ;
. FIG 17 : 16 illustre un schéma illustrant une partie du commutateur, notamment dans la poignée ;
. FIG. 18. : la figure 18 illustre une pluralité de configurations alternatives que peut prendre la station de mesure, notamment grâce au commutateur de figure 16 ou 17 ;
. FIG : 19 : la figure 19 illustre un procédé de sélection de configuration.

### Description détaillée

. Les figures 1 à 4 illustrent une représentation d'une station de mesure 100 selon au moins un mode de réalisation de la présente description. La station de mesure 100 se présente principalement sous la forme d'une base 102 sur laquelle un utilisateur peut poser ses pieds, par exemple à plat. L'utilisateur peut être sur la station de mesure ou assis sur une chaise. En position normale d'utilisation, les pieds de l'utilisateur sont posés à plat sur la station de mesure 100.L'épaisseur de la base 102 est par exemple inférieure à 10 cm, voire 6cm. La station de mesure 100 comprend un ou plusieurs capteurs 104 aptes à mesurer des informations physiologiques d'un utilisateur.

. Dans un mode de réalisation, certains capteurs 104 (par exemple des électrodes) sont montés sur un substrat 106 de la base 102, le substrat étant configuré pour recevoir les pieds d'un utilisateur. Le substrat peut être une plaque rigide, comme illustré sur les figures, et appelée plaque de mesure 106. La plaque de mesure 106 définit un plan parallèle à un plan XY. La plaque de mesure 106 peut être en verre. Néanmoins, le substrat peut être déformable sous le poids de l'utilisateur. Le substrat 106 peut être monté sur un socle 108, par exemple rigide, ou des pieds (non illustré). Dans le cas d'une base 102 fonctionnant comme un pèse-personne, des capteurs sont positionnés entre le substrat 106 et le socle 108 (architecture dite « sandwich ») ou entre le substrat 106 et les pieds (architecture dite « à pieds »). Les capteurs peuvent être des cellules de charge (généralement quatre) qui permettent, d'obtenir un poids, et donc une masse d'un utilisateur. Le socle 108 peut être en métal (aluminium, acier, etc.) ou en plastique.

. Comme visible en figure 2, la station de mesure 100 comprend de plus une plaque de support 202, qui peut être solidaire de la plaque de mesure 106. La plaque de support 202 est conçue pour recevoir une partie de l'électronique de la station de mesure 100, notamment via un circuit imprimé PCB (« *printed circuit board* ») monté sur la plaque de support 202. La plaque de support 202 est donc positionnée entre le socle 108 et la plaque de mesure 106.

. Dans une architecture à pieds, on définit deux groupes en déplacement l'un par rapport à l'autre : les pieds d'une part (groupe fixe), et tout le reste d'autre part (groupe mobile). Les cellules de charges relient mécaniquement ces deux groupes. La plaque de support, si présente, est alors généralement cachée par un capot externe solidaire de la plaque de mesure. Visuellement, seule la partie mobile est généralement visible.

. Dans une architecture sandwich, on définit deux groupes en déplacement l'un par rapport à l'autre : le socle 108 (et éléments associés) d'une part (groupe fixe), et tout le reste d'autre part (groupe mobile). Les cellules de charges relient mécaniquement ces deux groupes. Visuellement, les deux groupes sont généralement visibles.

. Dans un mode de réalisation, la station de mesure 100 comprend en outre une poignée 110, apte à être saisie par une moins une main de l'utilisateur, illustrée en figures 3 et 4. La poignée 110 peut être reliée à la station de mesure par un câble 302 (visible sur la figure 3). Afin d'avoir une station de mesure 100 pratique et sans câble volant, le câble 302 peut se déployer et se rétracter (par exemple s'enrouler et se dérouler) à l'intérieur de la base 102. A cette fin, un enrouleur (non visible sur les figures) est agencé dans un espace prévu entre le substrat 106 et le socle 108. Au moins deux positions sont ainsi définies pour la poignée : une position rangée (visible en figures 1 et 2) et une position déployée (visible en figure 3). La base 102 comprend en outre un support de poignée 112 qui peut accueillir la poignée 110 en position rangée. Le support de poignée 112 est par exemple monté sur le substrat 106. Il sera décrit plus en détail par la suite. La poignée 110 comprend elle aussi au moins un capteur 402.

. La poignée 110 est utilisée pour effectuer au moins une mesure parmi les mesures suivantes : ECG (ECG-1-voie entre les deux mains ou plusieurs voies avec d'autres membres), BIA (dites « segmentales »), IPG, éventuellement ESC. Les capteurs de la poignée 110 sont choisis notamment parmi : capteur optique pour PPG et électrodes.

. La base 102 peut comprendre un affichage 114 (par exemple un écran ou un affichage à LED ou à encre électronique) pour afficher des informations à destination de l'utilisateur. L'écran 114 est affiché en pointillé sur la figure 1 car, dans l'exemple des figures, il n'est pas ou peu visible lorsqu'il est éteint.

. Le socle 108 de la base 102 peut comprendre un chanfrein 204 pour faciliter la préhension de la station de mesure 100 lorsqu'elle est sur le sol.

. Dans un mode de réalisation, la base 102 a une forme essentiellement rectangulaire dans un plan XY. La base 102 a par exemple une forme essentiellement parallélépipédique dans l'espace XYZ.

. Lorsque la station de mesure 100 est positionnée à plat, la plaque de mesure 106 est parallèle à un plan XY. La station de mesure 100 comprend une direction longitudinale dans un plan XY et une dimension transversale dans un plan XY et orthogonale à la direction longitudinale. Par hauteur, on entend la dimension selon l'axe Z (aussi appelé épaisseur) ; par largeur, il est signifié la dimension transversale selon l'axe X ; par longueur, il est signifié la dimension longitudinale selon l'axe Y. En utilisation normale, les pieds de l'utilisateur se positionnent le long de la longueur Y de la base 102. Le bord de la station de mesure 110 (ou de la base 102, ou bien encore de la plaque de mesure 106) qui est le plus proche de la partie antérieure du pied en utilisation normale (c'est à dire les doigts de pied) est appelé bord antérieur et le bord opposé de la station de mesure 110 (ou de la base 102, ou bien encore de la plaque de mesure 106), qui est le plus proche de la partie postérieure du pied en utilisation normale (c'est-à-dire le talon) est appelé bord postérieur. On peut définir un axe médian, selon la longueur Y (longitudinale donc), autour duquel la plaque de mesure 106 est symétrique et qui permet de définir une partie gauche, destinée au pied gauche, et une partie droite, destinée au pied droit. La largeur X102 de la base 102 peut être comprise entre 330 et 400mm (par exemple environ 357mm) et la longueur de la base Y102 peut être comprise entre 300 et 360mm (par exemple environ 325mm). La longueur et/ou la largeur de la plaque de mesure 106 peuvent être légèrement moindres que celles du socle 108, de sorte que la plaque de mesure 106 soit légèrement en retrait par rapport au socle 108. Dans ce cas, la longueur et la largeur du socle 108 correspondent respectivement à la longueur et à la largeur données ci-dessus pour la base 102. Une telle conception permet de protéger la plaque de mesure 106 des chocs et des contacts avec l'environnement extérieur. La demande FR2106653, incorporée par référence, décrit une telle solution.

. Concrètement, comme illustré en figure 2 : la hauteur H102 de la base 102 peut être comprise entre 20 et 35mm (par exemple entre 35 et 40mm) et la hauteur maximale H100 de la station de mesure 100 peut être comprise entre 45 et 55mm (par exemple 51mm). Comme illustré sur les figures, seuls le support de poignée 112 et la poignée 110 sont en saillie selon la direction Z par rapport à la plaque de mesure 106. En détaillant les différentes dimensions : la hauteur Z108 du socle 108 peut être comprise entre 10 et 20mm (par exemple 18mm), la hauteur Z202 de la plaque de support 202 peut être comprise entre 3 et 6mm (par exemple 4mm), la hauteur Z106 de la plaque de mesure 106 peut être comprise entre 4 et 8mm (par exemple 6mm).

. Le câble 302 peut avoir une longueur comprise entre 50cm et 120cm. La longueur est choisie pour que la plupart des utilisateurs puisse saisir la poignée en se tenant debout et avoir les mains vers le bas (au repos).

. La station de mesure 100 pourrait toutefois avoir des formes et/ou des dimensions différentes, pourvu que la forme et/ou les dimensions permettent l'obtention des mesures présentement décrites. En particulier, la base 102 pourrait avoir une forme ovale ou plus arrondie dans le plan XY.

. La station de mesure 100 peut avoir une masse comprise entre 3 et 6kg (par exemple entre 4 et 5kg).

. Grâce au(x) capteur(s) de la base 102 et/ou au(x) capteur(s) de la poignée 110, la station de mesure 100 peut effectuer un groupe de mesures sur l'utilisateur. En particulier, les capteurs 104 utilisés comprennent des électrodes qui sont formées à partir de chemins conducteurs d'électricité montés sur le substrat 106 et/ou la poignée 110 (inserts métalliques, dépôts métalliques, etc.). Certaines mesures peuvent ne nécessiter que des capteurs de la base 102, d'autres mesures peuvent ne nécessiter que des capteurs de la poignée 110, d'autres mesures peuvent nécessiter simultanément des capteurs de la poignée 110 et de la base 102.

. La station de mesure 100 peut ainsi effectuer un ECG à l'aide de la poignée 110 (par exemple un ECG 1 voie), ou un ECG à l'aide de la poignée 110 et de la base 102 (par exemple un ECG multi-voie, comme un ECG six-voies). La station de mesure 100 peut ainsi effectuer une analyse d'impédancemétrie corporelle BIA à l'aide de la poignée 110 et/ou de la base (BIA entre les jambes et/ou BIA segmentale). La station de mesure peut ainsi effectuer un IPG dans l'arc de jambes (« *between legs* ») ou IPG dans le pied (« *in the foot* »).

. Les capteurs peuvent comprendre des électrodes aptes : à mesurer et/ou appliquer une tension (continue ou alternative) et/ou un potentiel (continu ou alternatif), et/ou injecter et/ou récupérer un courant (continu ou alternatif). Les fonctions de ces électrodes peuvent être choisies parmi la liste suivante : i+ et i-, pour l'injection de courant alternatif dans le corps d'un utilisateur ; V+ et V- pour mesurer une différence de potentiel dans le corps d'un utilisateur ; RA, LA et LL, pour mesurer une courant électrique traversant le corps d'un utilisateur ; sudo_cath, sudo_an, pour mesurer une conductivité de la peau et sudo_HiZ pour fixer le corps d'un utilisateur à une impédance donnée. Les électrodes i+ et i-, V+ et V- sont utilisées pour un BIA, un IPG ou un ICG ; les électrodes RA, LL, LL sont utilisées pour un ECG ; les électrodes sudo_cath, sudo_an et sudo_HiZ sont utilisées pour un ESC.

. En particulier, la station de mesure 100 est configurée pour réaliser différentes mesures. Le nombre d'électrodes étant limité (pour des considérations de surface et de nombre), la station de mesure 100 présente un agencement particulier d'électrodes, avec un commutateur.

. Comme indiqué précédemment, les capteurs peuvent comprendre des cellules de charge, grâce auxquelles la station de mesure 100 peut mesurer un poids et effectuer un BCG.

. La poignée 110 est illustrée en détail en figure 4. La poignée 110 permet à la station de mesure 100 de réaliser une plus grande variété de mesures ou bien des mesures plus complètes, grâce à une connexion électrique avec au moins une main, voire les deux mains. En particulier, la BIA segmentale et/ou l'ECG multi-voies sont rendus possibles par l'ajout de la poignée 110 à la base 102. Les capteurs 402 de la poignée 110 comprennent par exemple des électrodes aptes à mesurer et/ou appliquer une tension et/ou un potentiel et/ou injecter et/ou récupérer un courant.

. Dans un mode de réalisation, la poignée 110 comprend quatre électrodes, agencées en deux paires : une paire pour la main gauche et une paire pour la main droite. A cet effet, on nomme les électrodes de la poignée par : électrodes LH1, LH2, côte à côte sur une partie gauche de la poignée 110 et électrodes RH1, RH2, côte à côte sur une partie droite de la poignée (par partie gauche, respectivement droite, il est entendu la partie de la poignée destinée à être au contact de la main gauche, respectivement droite). « Côte à côte » signifie ici avec un espace entre les électrodes, pour isoler les électrodes les unes des autres. Les électrodes sont donc disposées à la suite les unes des autres entre deux extrémités de la poignée 110. Lorsque la poignée 110 est rectiligne, les électrodes sont disposées à la suite le long de la direction principale de la poignée 110. Les électrodes LH1 et RH1 sont positionnées axialement du côté d'une extrémité de la poignée ; les électrodes LH2, RH2 sont positionnées axialement du côté du centre de la poignée. On a donc, dans l'ordre, les électrodes suivantes : LH1, LH2, RH2, RH1.

. Dans un mode de réalisation, les capteurs 402 de la poignée 110, lorsqu'ils sont des électrodes sont réalisés sous la forme de plusieurs inserts métalliques dans la poignée 110. Parmi les matériaux utilisables pour les inserts métalliques, on peut citer l'acier inoxydable, le titane, le laiton, l'ITO (oxyde d'indium-étain), nickel (ou alliage de nickel), ou des plastiques conducteurs. Pour le traitement et/ou l'acquisition de signaux, en particulier de l'ECG, la poignée 110 peut comprendre de l'électronique de traitement (amplificateur opération en suiveur, etc.), notamment pour l'ECG et/ou l'impédancemétrie. Il est généralement préférable d'amplifier le signal au plus près des électrodes car le câble peut capter du bruit ambiant.

. La figure 5 illustre une vue schématique de l'architecture globale 500 dans laquelle la station de mesure 100 peut être insérée. Cette architecture globale forme un système comprenant la station de mesure 100. En particulier, la station de mesure 100 peut communiquer avec des dispositifs tiers via un réseau de communication 510, qui est par exemple un réseau sans-fil (notamment un réseau compatible avec au moins l'un des protocoles de communication suivants : Bluetooth, Wi-Fi, Ethernet, etc.). Les dispositifs tiers peuvent comprendre un serveur 520 et un terminal mobile 530 (smartphone, etc.). Le serveur 520 peut comprendre une circuiterie de contrôle 522, incluant un processeur 524 et une mémoire 526, et une interface entrée/sortie (« *input*/*output* », I/O) 528, qui permet à la circuiterie de contrôle de recevoir et d'envoyer des données vers le réseau de communication 510. Le terminal mobile 530 peut comprendre une circuiterie de contrôle 532, incluant un processeur 234 et une mémoire 236, un comprendre une interface entrée/sortie (« *input*/*output* », I/O) 538, qui permet à la circuiterie de contrôle de recevoir et d'envoyer des données. Le serveur 520 est un serveur distant, par exemple situé dans un centre de données (« *data center* » en anglais). Le terminal mobile 530 comprend en outre une interface d'utilisateur 540 (« *user interface* », UI) configurée pour afficher des informations à l'utilisateur et lui permettre, le cas échant, d'entrer des informations (comme la taille, le sexe, etc.). En particulier, la circuiterie de contrôle 532 est configurée pour faire fonctionner une application gérant l'environnement de la station de mesure 100. Le terminal mobile 530 est un objet personnel de l'utilisateur, généralement à proximité de ce dernier.

. La station de mesure 100 peut communiquer avec le serveur 520 et/ou le terminal mobile 530. Dans un mode de réalisation, la station de mesure 100 peut communiquer directement avec le terminal mobile 530, par exemple via Bluetooth ou Bluetooth Low Emission (BLE). Cette communication peut être mise à en œuvre à l'installation de l'appareil de mesure 100, notamment pour l'appairer avec le terminal mobile 530 et/ou pour configure une connexion au serveur 520 qui ne transite pas par le terminal mobile 530 et/ou en en secours d'une communication avec le serveur 520 défaillante. Dans un mode de réalisation, la station de mesure 100 peut communiquer directement avec le serveur 520, sans transiter par le terminal mobile 530. Cette communication permet à l'utilisateur d'utiliser la station de mesure même sans avoir son terminal mobile 530 à proximité.

. La station de mesure 100 comprend elle aussi une circuiterie de contrôle 550 avec un processeur 552 et une mémoire 554, et une interface entrée/sortie (« *input*/*output* », I/O) 556, qui permet notamment à la circuiterie de contrôle de recevoir et d'envoyer des données au réseau de communication 510. Le processeur 552 est configuré pour notamment traiter des données obtenues par les capteurs 104. En particulier, le processeur 552 peut exécuter des instructions d'un programme stocké dans la mémoire 554. La circuiterie de contrôle 550 peut comprendre un microcontrôleur, qui intègre le processeur 552, la mémoire 554 et l'interface entrée/sortie 556. La circuiterie de contrôle 550 peut en outre comprendre un dispositif frontal analogique (« *Analog Front End* », AFE). La circuiterie de contrôle 550 peut aussi comprendre un convertisseur analogique/digital (« *Analog to Digital Converters* », ADC). La station de mesure 100 comprend une source de tension (par exemple continue) 558 et une source de courant 560 (par exemple alternatif). La station de mesure 100 comprend aussi un voltmètre 562 (ou tout système permet de mesurer une tension). Le voltmètre 562 peut être intégré à l'AFE. La source de courant 560 peut être intégré à l'AFE et la source de tension 558 peut être intégrée au microcontrôleur MCU (par exemple via un convertisseur digital/analogique, « *digital to analog converter* » DAC). Certains capteurs 104 (en particulier les capteurs 402 de la poignée 110 de la figure 4 ou les électrodes 602 de la base 102 sur la figure 6) sont reliés à la circuiterie de contrôle 550 (par exemple au MCU ou à l'AFE). La station de mesure 100 comprend une batterie 564, apte à alimenter en énergie les différents composants de la station de mesure 100.

. La circuiterie de contrôle 550 et d'autres composants électroniques peuvent être montés sur un circuit imprimé PCB (« *printed circuit board* »), par exemple attaché à la plaque de support 202. Des connecteurs relient les chemins conducteurs d'électricité de la plaque de mesure au PCB. Afin de pouvoir modifier les connexions des électrodes aux différents composants de la station de mesure 100, la station de mesure comprend un commutateur 566. Le commutateur 566, qui peut comprendre une pluralité d'interrupteurs piloté par le MCU, sera décrit plus en détail par la suite).

. La circuiterie de contrôle 550 comprend par exemple un système d'acquisition d'ECG, un système d'impédancemétrie (pour BIA ou IPG), un système d'ESC (pour l'ESC). Pour chacun de ces systèmes, différents composants de la station de mesure 100 sont sollicités. Par exemple, le système d'acquisition d'ECG comprend des électrodes (représentées par 602 sur la figure 6 et 402 sur la figure 4) et un circuit électrique ECG 568 (qui intègre notamment différents étages d'amplification et/ou de filtrage et un démodulateur) ; le système d'impédancemétrie comprend notamment des électrodes (représentées par 602 sur la figure 6 et 402 sur la figure 4), la source de courant 560, le voltmètre 562 et un circuit électrique d'impédancemétrie 570 qui relie les électrodes à la source de courant et au voltmètre (qui intègre différents étages d'amplification et/ou de filtrage) : le système d'ESC comprend des électrodes, la source de tension 558 et un circuit électrique d'ESC 572 (qui intègre différents composants électroniques, dont des résistances). Le commutateur 566 permet de relier les électrodes notamment aux différents circuits 568, 570, 572 précités, ou bien de déconnecter toutes les électrodes de la circuiterie de contrôle 550.

. La circuiterie de contrôle 550 se trouve essentiellement dans la base 102, à l'exception de notamment quelques composants (amplification, filtrage et interrupteurs) disposés dans un PCB dans la poignée 110, pour traiter les signaux avant de les faire transiter par le câble 302.

. Comme indiqué auparavant, la station de mesure 100 comprend aussi l'affichage 114, telle qu'un écran (OLED/PMOLED, Retina, etc.), pour afficher à l'utilisateur des informations. Alternativement, la station de mesure 100 ne comprend pas d'affichage.

. Dans un mode de réalisation, les capteurs 104 incluent des chemins conducteurs d'électricité 602 (appelés « électrodes ») sur la base 102 (voir notamment figures 6 et 7). Les électrodes 602 peuvent prendre la forme d'un dépôt métallique sur une face supérieure 604 de la plaque de mesure 106. La face supérieure 604 de la plaque de mesure 106 est définie comme la face recevant les pieds de l'utilisateur (la face externe visible). Pour assurer la connexion électrique avec le PCB, les électrodes 602 passent par un bord de la plaque de mesure 106 et s'étendent jusque sur une face inférieure 702 de la plaque de mesure 106. Le bord (ou les bords concernés) de la plaque de mesure 106 peut présenter une forme arrondie pour assurer que le dépôt métallique se fasse bien et que la continuité électrique soit assurée. De plus, un bord arrondi permet d'éviter les risques de blessure lors de la préhension de la station de mesure 100. Par arrondi, il est signifié un arc de cercle ou des formes similaires. Le bord arrondi permet aussi de simplifier le dépôt métallique lors de la fabrication. La demande FR2106653, incorporée par référence, décrit en détail ces chemins conducteurs d'électricité.

. Les électrodes 602 sont connectées au PCB via un connecteur, qui permet de faire la connexion entre le chemin de conduction électrique sur la face inférieure 702 et le PCB monté sur la plaque de support 202. Le commutateur 566 permet de connecter et déconnecter les électrodes aux différents systèmes (système d'acquisition ECG, système d'impédancemétrie, système d'ESC, etc.). De la sorte, chaque électrode peut avoir plusieurs fonctions différentes en fonction de la position de commutation du commutateur 566. Le commutateur 556 comprend par exemple une pluralité d'interrupteurs pilotés par le MCU.

. La face supérieure 604 de la base 102 comprend un groupe gauche LG d'électrodes (destiné à être en contact avec le pied gauche, et un groupe droit RG d'électrodes destiné à être en contact avec le pied droit. Lorsque la base 102 est posée en condition normale d'utilisation, l'utilisateur met ses pieds sur une partie gauche de la balance et une partie droite de la balance (les doigts de pied étant du côté de l'affichage 114). Les figures 6 et 7 représentent des chemins conducteurs d'électricité L1, L3, L5, L7, L9, L11, L13, L15, L17 qui forment les électrodes du groupe gauche LG d'électrodes et des chemins conducteurs d'électricité R2, R4, R6, R8, R10, R12, R14, R16, R18, qui forment les électrodes du groupe droite RG d'électrodes.

. Les électrodes 602 peuvent prendre la forme de bandes parallèles les unes aux autres le long de la direction X (les bandes s'étendent le long de la largeur X de la base 102).

. Dans l'architecture illustrée, les couples de chemins conducteurs d'électricité L1 et L3 ; L15 et L17 ; R2 et R4 ; R16 et R18 ne sont pas indépendants mais sont reliés électriquement en permanence, de sorte que la base 102 comprend en pratique sept électrodes indépendantes dans le groupe gauche LG et sept électrodes indépendantes dans le groupe droit RG. Ces connexions électriques permanentes peuvent se faire via les chemins conducteurs d'électricité sur la plaque de mesure 106 (par exemple sur la face inférieure 702, non-illustré) ou via le PCB de la station de mesure 100.

. Dans un exemple, les chemins conducteurs d'électricité de la face supérieure 604 correspondant aux électrodes 1301-1312 ont une dimension (sur la face supérieure 604) selon la longueur Y comprise entre 1,5cm et 2 cm (par exemple 1,7cm) ; l'espacement entre deux bandes peut être compris entre 0,5cm et 1cm (par exemple 0,85cm) ; les électrodes peuvent avoir une dimension selon la largeur X supérieure à 10cm.

. En particulier, chaque groupe LG, RG peut comprendre au moins quatre électrodes indépendantes pour notamment pouvoir effectuer un IPG dans le pied (deux électrodes connectées à la source de courant alternatif 560 et deux électrodes connectées au voltmètre 562). Dans un autre mode de réalisation, chaque groupe LR, RG peut comprendre au moins deux électrodes indépendantes (pour effectuer un ESC avec anode/cathode et une électrode haute impédance, ou pour effectuer un BIA ou un IPG entre les jambes), ou trois électrodes indépendantes.

. Dans un mode de réalisation, la station de mesure 100 comprend une pluralité de surfaces conductrices (les chemins conducteurs d'électricité décrits précédemment) permettant de mesurer la conductance de la peau (mesure ESC, pour notamment effectuer un ESC), notamment celle de la peau sous les pieds. Sur la figure 6, ces surfaces conductrices prennent la forme de bandes parallèles L1 à L17 et R2 à R18. En particulier, la base 102 comprend deux groupes de chemins conducteurs d'électricité (aussi appelés électrodes) : un premier groupe d'électrode LG, qui est positionné sur une partie gauche de la base 102 en utilisation normale (appelé groupe gauche, mais ce terme ne doit pas être interprété limitativement pour un pied gauche uniquement), et un deuxième groupe d'électrodes RD, qui est positionné sur une partie droite de la base 102 en utilisation normale (appelé groupe droite, mais ce terme ne doit pas être interprété limitativement pour un pied gauche uniquement). En particulier, un utilisateur pourrait monter à l'envers sur la base 102 (pied gauche sur le groupe droit RG et pied droit sur le groupe gauche LG). Les figures 1 et 6 illustrent un exemple d'un mode de réalisation des groupes droite et gauche LG, RG. Les deux groupes d'électrodes LG, RG sont espacés l'un de l'autre d'une distance comprise par exemple entre 0,1cm et 0,5cm.

. Les groupes gauche RG et droite RD d'électrodes sont formées par des électrodes montées sur la plaque de mesure 106. Sur le schéma simplifiée 800 de la figure 8 sont représentées quatre électrodes : les électrodes 801 et 803 pour le groupe gauche LG et les électrodes 802 et 804 pour le groupe droit RG. Les groupes RG, LG peuvent comprendre davantage d'électrodes, comme décrit par la suite.

. Sauf mention explicite de la poignée, l'intégralité des caractéristiques propres à la mesure de l'activité sudorale peut être effectuer avec la station de mesure 100 comprenant uniquement la base 102. On parlera donc de station de mesure 100 ou de base 102 indifféremment.

. Les groupes gauche et droit d'électrode LG, RG permettent d'effectuer un ESC, c'est-à-dire une mesure de l'activité sudorale des pieds. Les documents WO2006/136598, WO2008/107324, WO2013/075963, WO2014/033105, WO2015/036530 WO2016/083432 décrivent un système permettant d'effectuer un ESC. Le lecteur est renvoyé à ces documents pour la théorie et les principes physiologiques.

. La station de mesure 100 (la base 102) comprend un système d'ESC, apte à réaliser une mesure de la conductance électrochimique de la peau pour évaluer une fonction sudorale de celle-ci. En particulier, le système d'ESC comprend une source de d'excitation continue 806 (par exemple la source de tension 528 qui génère une tension continue ou un générateur de tension continue), adapté pour générer des signaux de tension continue et en particulier des paliers de tension constante. Chaque palier de tension peut durer entre 0,2s et 5s. La tension délivrée par la source de tension continue est par exemple comprise entre 0 et 10 V, voire entre 0 et 4 V.

. La source de tension continue 806 est connectée aux électrodes 801 à 804, par le commutateur 566 afin de pouvoir connecter ou déconnecter les électrodes à la source de tension continue 806. Dans le cas d'un ESC, les électrodes 801 à 804 sont connectées par paires à la source de tension continue 806 pour fonctionner respectivement en anode et cathode. En particulier, une électrode de chaque groupe LG, RG est connectée aux bornes (ou pôle, pour une source) de la source 806 pour former une paire d'anode et cathode (par exemple l'électrode 801 en anode et l'électrode 802 en cathode). Deux électrodes d'un même groupe LG, RG peuvent être connectée au même pole de la source 802 (par exemple, les électrodes 801, 803 en anode et les électrodes 801, 804 en cathode).

. Dans la présente description, il est possible d'inverser les polarités, par exemple avec le commutateur 566 ou via le générateur de tension 806 lui-même, de sorte qu'anode et cathode sont inversables. Par conséquent, lorsqu'il sera dit qu'une première électrode est reliée à la cathode et qu'une deuxième électrode est reliée à l'anode, il est aussi indiqué que l'anode et la cathode peuvent être inversées.

. La station de mesure 100 comprend en outre une unité de contrôle 810 (par exemple le MCU), apte à contrôler la source de tension continue 806 et à contrôler un circuit de mesure 812 d'un circuit d'ESC 572. Le circuit de mesure 812 permet notamment à l'unité de contrôle 810 de mesurer le courant traversant les pieds de l'utilisateur. A cette fin, l'unité de contrôle 810 peut mesurer une tension aux bornes d'une résistance dite résistance de mesure Rm. La résistance de mesure Rm peut être variable et pilotable. L'unité de contrôle 810 peut faire partie de la circuiterie de contrôle 550.

. Le commutateur 566 permet de sélectionner deux électrodes comme anode et cathode, la première étant reliée à la source de tension continue 806 et la seconde étant reliée à la résistance de mesure Rm.

. Le groupe gauche RG ou le groupe droite RD comprend au moins deux électrodes électriquement indépendantes 801, 803 et 802 et 804 les unes des autres. Par électriquement indépendantes les unes des autres, il est signifié que les deux électrodes 801, 803 ou 802, 804 ne sont pas en permanence électriquement connectées et qu'il est par conséquent possible d'avoir une configuration dans laquelle, comme lesdites deux électrodes sont électriquement indépendantes l'une de l'autre, le commutateur 566 peut leur attribuer deux fonctions différentes. Inversement, dans un mode de réalisation, le commutateur 566 peut relier électriquement les deux électrodes 801, 803 ou 802, 804, de sorte qu'elles aient la même fonction.

. Grâce à cette indépendance, la station de mesure 100 peut effectuer des mesures en ayant une électrode 801 à 804 (d'un même groupe LG, RG) qui peut prendre, à des moments différents, au moins deux fonctions différentes, en fonction des configurations permises par le commutateur 566. Par exemple, la station de mesure 100 peut effectuer séquentiellement (à la suite l'une de l'autre), grâce au commutateur 566, au moins deux mesures parmi : ESC, BIA, IPG, ICG, ECG (avec ou sans la poignée 110).

. Dans un mode de réalisation, le groupe gauche RG et le groupe RD comprennent chacun au moins deux électrodes 801, 803, 802, 804 électriquement indépendantes. De la sorte, chaque groupe RG et RD peut avoir plusieurs fonctions : en particulier, toute mesure qui nécessite d'avoir deux électrodes de fonctions différentes dans les deux groupes (IPG entre les jambes, BIA entre les jambes, IPG dans le pied, etc.).

. Plusieurs modes de réalisation illustrant une telle configuration vont être donnés.

. Dans une configuration, dite configuration à tension continue CC, au moins une des électrodes 801, 803 du groupe droit RG et/ou au moins une des électrodes 802, 804 du groupe gauche LG est connectée à la source tension continue 806 (électrode dite « active ») par le commutateur 566.

. Dans un mode de réalisation, en configuration CC, au moins une électrode de chaque groupe LG, RG n'est pas reliée à la source de tension continue 806 par le commutateur 566. Cette au moins une autre électrode dudit groupe peut être connectée en haute impédance (électrode dite « passive ») par le commutateur. La haute impédance est une valeur comprise entre 500 kOhms et 20 MOhms (par exemple 10 MOhms). L'électrode passive à haute impédance a pour rôle de permettre à l'unité de contrôle 810 de mesurer le potentiel du corps d'un utilisateur sans créer de fuite de courant vers ce dernier. Par exemple, le commutateur 566 relie l'électrode à un circuit comprenant une résistance de la valeur précitée (avec par exemple un amplificateur opérationnel).

. Dans un autre mode de réalisation, en configuration CC, toutes les électrodes du groupe LG ou RG sont reliées à la source de tension continue 806 par le commutateur 566. Cela permet d'avoir une surface maximale d'électrodes en anode et cathode. En particulier, dans ce mode de réalisation, toutes les électrodes du groupe gauche LG sont reliées à une borne de la source de tension continue 806 et toutes les électrodes du groupe droite LG sont reliées à l'autre borne de la source de tension continue 806. Afin de quand même pouvoir mesurer l'impédance du corps, la poignée 110 peut être connectée en haute impédance par le commutateur 566 (non illustré sur les figures). L'utilisateur tient alors la poignée lorsqu'il effectue un ESC.

. On définit plusieurs surfaces sur la face supérieure 604 de la plaque de mesure 106 : la surfaces Sa des électrodes qui sont des électrodes actives en configuration CC (surface cumulée de toutes ces électrodes), la surface St de toutes les électrodes qui sont utilisées en configuration CC (électrodes actives et électrodes passives). On définit en outre une surface efficace, qui est la surface des électrodes qui est effectivement en contact avec le pied. Pour affranchir les définitions de la notion de pied de l'utilisateur, on définit une surface efficace élargie Sr, qui peut correspondre à un rectangle virtuel de 30cm le long de la longueur Y et 15cm le long de largeur (il s'agit d'un rectangle à l'intérieur duquel un pied peut être posé). On définit aussi une surface Se de l'enveloppe convexe qui englobe les électrodes d'un même groupe LG, RG. Il s'agit de la plus petite surface convexe qui comprend toutes les électrodes d'un même groupe LG, RG

. Pour un groupe LG, RG, la surface de la ou des électrodes qui sont connectées à la source de tension continue 806 en configuration CC représente plus de 50% de la surface des électrodes du groupe LG, RG, voire plus de 75%, plus de 80%, plus de 85%, voire plus de 90% (ratio Sa/St). Cette condition de surface sera appelée « critère de surface » dans le reste de la description. Cela permet de maximiser la surface totale d'électrodes actives, qui permettent d'activer la fonction sudorale des pieds, afin d'améliorer la qualité de la mesure. De plus, la surface de la ou des électrodes qui sont connectées à la source de tension continue 806 en configuration CC représente plus de 50% de la surface de l'enveloppe convexe Se, voire plus de 55%, plus de 60%, voire plus de 80% (ratio Sa/Se). Cela permet d'assurer que les électrodes actives en configuration CC sont réparties sous le pied et pas localisées à un seul endroit. En effet, la distribution des glandes sudoripares peut varier entre individus et il est important de pouvoir limiter les effets de cette variation. De la même façon, cette condition permet de limiter les effets de placement du pied sur la base 102. Afin de s'assurer que la surface des électrodes soit suffisamment grande pour couvrir une amplitude importante du pied, la distance Dmax entre les deux électrodes extrémales (incluant les électrodes elles-mêmes) le long de la longueur Y peut être d'au moins 20 cm, voire 25 cm. De la même façon, chaque électrode s'étend le long de la largeur X sur au moins 10 cm, voire 15 cm. Alternativement ou complémentairement le ratio Se/Sr peut être préférablement supérieur à 75%, voire 90%, voire supérieure à 100%.

. Pour intégrer la fonction d'ESC dans la base 102 (donc sans poignée ou sans électrodes en contact avec une autre région que les pieds), une électrode de la base 102 est configurée pour être une électrode passive, en haute impédance. Cette connexion peut être permanente (par exemple via une connexion sur le PCB) ou activable et désactivable via le commutateur 566.

. Il existe en pratique au moins deux configurations CC pour toute paire d'électrodes : un configuration CC avec anode/cathode et une configuration CC avec cathode/anode.

. La surface des électrodes peut être calculée de plusieurs façons. Idéalement, seule la surface efficace des électrodes est prise en compte, c'est-à-dire la surface des électrodes qui est effectivement en contact avec le pied. On peut définir une surface efficace élargie qui correspond à une zone de la face supérieure de la base 102 où le pied est susceptible d'être posé en utilisation normale de la station de mesure 100. Enfin, on peut définir une surface totale, qui correspond à la surface totale des électrodes sur la face supérieure 604. Dans le cas des bandes illustrées en figure 6, qui ont toutes une forme identique, ces trois définitions sont identiques. Toutefois, il est possible d'envisager des configurations dans lesquelles la surface efficace est sans rapport avec la surface totale, mais la surface efficace élargie vérifie les critères mentionnés précédemment. A l'intérieur de ce rectangle, le critère de surface est vérifié.

. Dans le mode de réalisation illustré en figures 1 et en 6, en configuration CC, huit bandes sur neuf d'un groupe LG, RG sont connectées à la source 566 et une bande sur neuf du même groupe LG, RG est connectée en haute impédance (bande passive). La bande en électrode passive est préférablement choisie parmi L7 ou L9 (ou respectivement R8 ou R9) afin d'être au niveau de la voûte plantaire où le contact du pied avec la face supérieure 604 est plus faible qu'ailleurs.

. La base 102 de la station 100, avec ou sans poignée, est configurée pour effectuer plusieurs mesures, dont notamment des mesures d'impédancemétrie, comme du BIA ou IPG. A cette fin, la station de mesure 100 comprend un système d'impédancemétrie, apte à réaliser une mesure d'impédance du corps d'un utilisateur en réponse à une excitation électrique. En particulier, le système d'impédancemétrie peut comprendre une source d'excitation alternative 814 (par exemple la source de courant alternatif 600 par exemple ou un générateur de courant alternatif), adapté pour injecter un courant alternatif (par exemple sinusoïdale). Les valeurs de courant sont connues de l'homme du métier. La source de courant alternatif 814 est connectée aux électrodes 801 à 804 par le circuit électronique. Le commutateur 566 permet de connecter et déconnecter des électrodes 801 à 804 à la source de courant alternatif 814. Dans le cas d'une impédancemétrie, les électrodes 801, 804 sont connectées par paire à la source de courant alternatif 814, aux bornes négative et positive.

. Dans une configuration à courant alternatif, dite configuration AC, au moins une des électrodes 801 à 804 parmi le groupe droit RG et/ou le groupe gauche LG est connectée à la source de courant alternatif 814 par le commutateur 566. Plusieurs configurations AC sont possibles : notamment, pour une architecture d'électrodes données et de fonctions données, il peut exister plusieurs configurations AC pour effectuer du BIA et plusieurs configurations AC pour effectuer de l'IPG. Le commutateur 566 est configuré pour sélectivement activer ou désactiver la configuration AC et/ou pour alterner entre les différentes configurations AC. On parlera de configuration ACf (f pour « *feet* », pied) lorsque deux électrodes d'un même groupe LG, RG sont connectées aux deux bornes de la source de courant alternatif 814 car une telle configuration permet d'effectuer un IPG dans le pied ; on parlera de configuration ACb (b pour « *between the legs* », entre les jambes) lorsqu'une borne de la source de courant alternatif 814 est reliée à au moins une électrode d'un groupe LG, RG et l'autre borne de la source de courant alternatif 814 est reliée à un moins une électrode de l'autre groupe RG, LG, car cette configuration permet notamment de faire un IPG entre les jambes ou un BIA entre les jambes.

. Sauf exception explicitement mentionnée, toute configuration ACf décrite pour un groupe d'électrode LG, RG est applicable pour l'autre groupe d'électrodes RG, LG (symétrie entre les deux groupes). En revanche, la configuration ACf pied gauche n'est pas activée en même temps que la configuration ACf pied droit (pour des raisons liées aux composants).

. Le commutateur 566 peut être configuré pour sélectivement commuter entre au moins une des configurations CC et au moins une des configurations AC.

. Parmi les configurations AC, on peut citer une configuration où les deux bornes de la source de courant alternatif 814 sont connectées à au moins deux électrodes parmi les groupes gauche LG et droite RG : les configurations ACf (IPG dans le pied) et ACb (IPG arc-de-jambe ou BIA sans poignée) en font partie. Il existe aussi des configurations où une seule borne de la source de courant alternatif 814 est connectée à au moins une électrode parmi les groupes gauche LG et droite RG ; dans ce cas, la station de mesure 100 comprend la poignée 110 et l'autre borne est reliée à une électrode de la poignée (BIA segmentale notamment). Cette configuration sera appelée ACs (« s » pour segmentale car elle permet de faire une BIA segmentale).

. En configuration AC, l'unité de contrôle 810 peut avoir besoin de mesurer un potentiel au niveau du corps d'un utilisateur (pieds et/ou mains). Pour cela, la station de mesure 100 comprend un voltmètre 562 apte à mesurer une différence de potentiel entre deux électrodes. Le commutateur 566 permet de connecter un couple d'électrodes (par exemple parmi les électrodes 801 à 804, mais aussi parmi les électrodes de la poignée) aux bornes du voltmètre 562, afin de mesurer une différence de potentiel.

. En pratique, la ou les sources de courant peuvent ne pas avoir deux bornes telles que représentées sur les schémas de principe. Néanmoins, il y a quand même un pôle i+ ou i-, V+ ou V-qui sont déterminés par le circuit électrique. Le terme de « bornes » fait alors références à ces pôles.

. Différents agencements de chemins conducteurs d'électricité formant électrodes vont être présentés, en relation avec les figures 9 à 15. Chaque agencement est limité par des contraintes physiques : la disposition des chemins conducteurs d'électricité le long de la longueur Y doit permettre au pied d'être en contact avec lesdits chemins conducteurs d'électricité ; par conséquent, la distance maximale Dmax (voir sur les figures 9 à 15) entre les électrodes extrémales le long de la longueur Y dans un groupe LG, RG est limitée. Les agencements peuvent être symétriques autour d'un axe de direction Y.

. En configuration CC, la paire d'électrodes reliées à la source de tension continue 806 comprend au moins une électrode du groupe gauche LG et au moins une électrode du groupe droit RD. Par exemple, le groupe gauche LG contient la cathode et le groupe droit RG contient l'anode, ou inversement. En revanche, deux électrodes d'un même groupe LG, RG ne peuvent être reliées aux deux bornes de la source de tension continue 806 (au sein d'un même groupe LG, RG, il ne peut y avoir simultanément une électrode fonctionnant en cathode et une autre électrode fonctionnant en anode).

. Sur les figures 9 à 15, « HiZ » signifie que, dans une configuration CC, l'électrode est reliée à la haute impédance ; « An » ou « Cath » signifie que, dans une configuration CC, l'électrode est reliée à la source de tension continue 806 (l'un ou l'autre des bornes, respectivement) ; i+ ou i-signifie que dans une configuration AC, l'électrode est reliée à la source de courant alternatif 814 (l'un ou l'autre des bornes, respectivement), V+ ou V- signifie que, dans une configuration AC, l'électrode est reliée au voltmètre 562 (l'un ou l'autre des bornes, respectivement) ; « - » signifie que, quelle que soit la configuration, l'électrode est inactivée (par exemple non connectée électriquement, déconnectée par le commutateur 556). Une même électrode pourra donc avoir plusieurs de ces symboles, puisqu'elle peut, en fonction de la configuration déterminée par le commutateur 566, séquentiellement assurer plusieurs fonctions.

. La figure 9 illustre un agencement 900 dans laquelle chaque groupe LG, RG comprend deux électrodes indépendantes 901, 903 (groupe LG) et 902, 904 (groupe RG). Les fonctions des électrodes indiquées sur la figure 9 sont sous la forme « CC/ACb ».

. En configuration CC, le commutateur 566 connecte les électrodes 901 à la source de tension continue 806 (anode/cathode ou cathode/anode). Dans une variante (non illustrée), les électrodes 903 et 904 sont elles aussi reliées à la source de tension continue 806 (électrode 901 avec l'électrode 903 et l'électrode 902 avec l'électrode 904). Dans une variante (illustrée), le commutateur 566 relie les électrodes 903 et 904 à la haute impédance (HiZ). Dans l'exemple illustré, les électrodes passives 903 et 904 sont situées, le long de la longueur Y, sous les électrodes 901, 902. Néanmoins, dans cette configuration, il existe un risque que le pied de l'utilisateur ne soit pas au contact des électrodes 903 et 904. La superficie des électrodes 901, 903, 902, 904 est choisie de sorte que la surface des électrodes actives en configuration CC vérifient le critère de surface mentionné précédemment.

. En configuration AC, le commutateur 566 relie au moins une électrode 901, 902, 903, 904 à la source de courant alternatif 814. En particulier, en configuration ACb, le commutateur 566 relie la paire d'électrodes 901 et 902 aux bornes du voltmètre 562 et le commutateur 566 relie la paire d'électrodes 903 et 904 aux bornes de la source de courant alternatif 814. L'agencement 900 permet de faire un BIA entre les jambes ou un IPG entre les jambes.

. Dans le cas d'une configuration ACs (BIA segmentale) avec la station 100 qui inclut la poignée 110, certaines des électrodes du groupe gauche LG ou le groupe droit RG peuvent être connectées à une borne de la source de courant alternatif 814 ou au voltmètre 562 sans que d'autres électrodes du même groupe LG, RG ne soit connectées à l'autre borne de la source de courant alternatif 814 ou au voltmètre 562.

. La configuration 900 permet ainsi, sur une base 102, d'effectuer un ESC qui couvre la majeure partie du pied (avec notamment une séparation pied droit pied gauche possible grâce à l'électrode haute impédance), tout en ayant une base 102 polyvalente qui peut effectuer de l'impédancemétrie entre les jambes (BIA, IPG). Le commutateur 566 permet de basculer d'une configuration CC à une configuration AC.

. Toutefois, un tel agencement peut présenter des inconvénients, notamment en ce qui concerne le positionnement du pied, qui peut ne pas être au contact des électrodes 903, 904. Néanmoins, agrandir ces électrodes signifie que la surface des électrodes 901, 902 pour le ESC est réduite, ce qui n'est pas souhaitable. En outre, le nombre de mesures effectuables est limité (par de configuration ACf par exemple, pour faire un IPG dans le pied).

. La figure 10 illustre un agencement 1000 ne présentant pas les inconvénients précités. Chaque groupe LG, RG comprend trois électrodes indépendantes 1001, 1003, 1005 (LG) et 1002, 1004, 1006 (RG). Les fonctions des électrodes indiquées sur la figure 10 sont sous la forme « CC/ACb ».

. Dans cet agencement, l'électrode 1003 (respectivement 1004) est située, le long de la longueur Y, entre les électrodes 1001, 1005 (respectivement 1002, 1006).

. En configuration CC, le commutateur 566 relie les électrodes 1001, 1005 (LG) et 1002, 1006 (RG) à la source de tension continue 806 (anode/cathode ou cathode/anode). Dans une variante non illustrée, les électrodes 1003 et 1004 sont elles aussi reliées à la source de tension continue 806 (électrode 1003 avec les électrodes 1001 et 1005 et l'électrode 1004 avec les électrodes 1002 et 1006) ; dans une variante illustrée, le commutateur 566 relie les électrodes 1003 et 1004 à la haute impédance HiZ.

. La superficie des électrodes est choisie de sorte que la surface des électrodes actives en configuration CC vérifie le critère de surface mentionné précédemment. Cela signifie que la surface de l'électrode passive (haute impédance) est plus petite que la surface d'un électrode active. En particulier, dans cet agencement, les électrodes 1001, 1005, 1002, 1006 peuvent être suffisamment grande pour que Se/Sr soit supérieur à 100%. Dans un exemple, les électrodes 1001, 1002, 1005, 1006 ont une dimension selon la longueur Y d'au moins 10 cm (par exemple 14cm) ; les électrodes 1004 ont une dimension selon la longueur Y compris entre 1,5cm et 2,5cm ; les électrodes peuvent avoir une dimension selon la largeur X supérieure à 10cm.

. En configuration ACb, le commutateur 566 relie les électrodes 1001 et 1002 au voltmètre 562 et le commutateur 566 relie les électrodes 1005 et 1006 à la source de courant alternatif 814. La connexion entre la borne négative et la borne positive peut être inversée. En configuration ACb, les électrodes 1003, 1004 sont inactives. L'agencement 1000 permet de faire un BIA entre les jambes ou un IPG entre les jambes.

. La configuration 1000 permet ainsi, sur une base 102, d'effectuer un ESC qui couvre la majeure partie du pied (avec notamment une séparation pied droit pied gauche possible grâce à l'électrode haute impédance), tout en ayant une base 102 polyvalente qui peut effectuer de l'impédancemétrie. Du fait du positionnement des électrodes 1003, 1004 au milieu du pied et du fait de la possibilité d'avoir des électrodes 1001, 1005 de grande taille, la sensibilité à la position du pied sur la longueur Y est assez faible, ce qui assure une bonne répétabilité des mesures.

. Un tel agencement est particulièrement adapté lorsque l'on ne souhaite pas avoir de configuration ACf, c'est-à-dire qu'on ne souhaite pas effectuer d'IPG dans le pied.

. La figure 11 illustre un agencement 1000 ne présentant pas les inconvénients précités. Chaque groupe LG, RG comprend quatre électrodes indépendantes 1101, 1103, 1105, 1107 et 1102, 1104, 1106, 1108. Les fonctions des électrodes indiquées sur la figure 11 sont sous la forme « CC/ACf (droit et gauche)/ACb ». Les agencements ACf droite et gauche ne sont pas mis en œuvre simultanément (pour des questions d'acquisition des données par la circuiterie de contrôle et de commutateur).

. En configuration CC, le commutateur 566 relie les électrodes 1101, 1105, 1107 (LG) et 1102, 1106, 1108 (RG) à la source de tension continue (anode/cathode ou cathode/anode). Dans une variante non illustrée, les électrodes 1103 et 1104 sont elles aussi reliées à la source de tension continue 806 (reliées à la même borne que les autres électrodes de leur groupe d'électrodes) ; dans une variante illustrée, le commutateur 566 relie les électrodes 1103 et 1104 à la haute impédance HiZ. Les électrodes 1103 et 1105 (respectivement 1104 et 1106) peuvent être inversées.

. La superficie des électrodes est choisie de sorte que la surface des électrodes actives en configuration CC vérifie le critère mentionné précédemment. En particulier, si les électrodes 1101 à 1108 ont toutes la même surface, le ratio de la surface des électrodes actives sur la surface totale est de 75%.

. Plusieurs configurations AC sont possibles. Dans une configuration ACf, le commutateur 566 relie les électrodes 1101 et 1107 aux bornes à la source de courant alternatif 814 et le commutateur 566 relie les électrodes 1103 et 1105 aux bornes du voltmètre 562. Cette configuration permet de faire un IPG dans le pied.

. Dans une configuration ACb, le commutateur 566 relie une ou deux parmi les électrodes 1101, 1103 et une ou deux parmi les électrodes 1102, 1104 aux bornes du voltmètre 562 et relie une ou deux parmi les électrodes 1105 et 1107 et une ou deux parmi les électrodes 1106 ou 1108 aux bornes de la source de courant alternatif 814. Cette configuration ACb permet de faire un IPG en arc-de-jambe ou un BIA entre les jambes.

. Dans le cas d'une configuration ACs (BIA segmentale) avec la station 100 qui inclut la poignée 110, certaines des électrodes du groupe gauche LG ou le groupe droit RG peuvent être connectées à une borne de la source de courant alternatif 814 ou au voltmètre 562 sans que d'autres électrodes du même groupe LG, RG ne soit connectées à l'autre borne de la source de courant alternatif 814 ou au voltmètre 562.

. L'agencement 1100 permet ainsi de faire un BIA segmental, un BIA entre les jambes, un IPG entre les jambes et un IPG dans le pied.

. L'agencement 1100 permet ainsi, sur une base 102, d'effectuer un ESC qui couvre la majeure partie du pied (avec notamment une séparation pied droit pied gauche possible grâce à l'électrode haute impédance), tout en ayant une base 102 polyvalente qui peut effectuer de l'impédancemétrie avec un haut degré d'adaptabilité.

. Toutefois, en configuration ACf (pour IPG dans le pied), l'agencement 1100 propose deux électrodes 1103 ou 1105 (ou 1104 et 1106) qui sont reliées aux deux bornes de la source de courant alternatif 814 tout en étant assez proche l'une de l'autre (distance Δ sur les figures, qui est défini comme la distance minimale entre les électrodes V+ et V- en configuration ACf). Cette proximité peut générer une perte de qualité du signal dans l'obtention de la tension par le voltmètre 562. Il peut alors être envisagé d'espacer les électrodes 1103 et 1105 entre elles (et 1104 et 1106 entre elles), mais, alors, du fait de la contrainte de la distance Dmax, la taille des électrodes 1101 à 1108 doit diminuer. En configuration CC, cela signifie que la surface des anodes ou cathodes en contact avec le pied diminue, ce qui génère des difficultés pour la mesure d'ESC.

. La figure 12 illustre un agencement 1200 qui vient pallier la difficulté précitée au sujet de l'agencement 1100. Chaque groupe LG, RG comprend cinq électrodes indépendantes 1201, 1203, 1205, 1207, 1209 et 1202, 1204, 1206, 1208, 1210. Les fonctions des électrodes indiquées sur la figure 12 sont sous la forme « CC/ACf (droite et gauche)/ACb ». Les agencements ACf droite et gauche ne sont pas mis en œuvre simultanément (pour des questions d'acquisition des données par la circuiterie de contrôle et de fonctionnement du commutateur).

. En configuration CC, le commutateur 566 relie les électrodes 1201, 1203, 1207, 1209 et 1202, 1204, 1208, 1210 à la source de tension continue 806 (anode/cathode ou cathode/anode). Dans une variante non illustrée, les électrodes 1205 et 1206 sont elles aussi reliées à la source de tension continue 806 (reliées à la même borne que les autres électrodes de leur groupe d'électrodes) ; dans une variante illustrée, le commutateur 566 relie les électrodes 1205 et 1206 à la haute impédance HiZ. L'électrode 1205 (respectivement 1206) peut être inversée avec l'électrode 1203 ou 1207 (respectivement 1206 avec 1204 ou 1208).

. La surface des électrodes est choisie de sorte que la surface des électrodes qui sont actives en configuration CC vérifie le critère de surface mentionné précédemment. La configuration avec cinq électrodes indépendantes permet de vérifier le critère de surface mentionné précédemment sans compromettre la possibilité d'effectuer une pluralité de mesures différentes (ESC, BIA, IPG, etc.) ni la qualité des mesures. En particulier, si les électrodes 1201 à 1210 ont toute la même surface, le ratio de la surface des électrodes actives sur la surface totale est de 80%. Si les électrodes 1205, 1206 (électrodes configurées pour être passive en configuration CC) sont plus petites (comme illustré), ce ratio augmente.

. Comme pour l'agencement 1100, la configuration ACf est possible car chaque groupe d'électrode LG, RG comprend au moins quatre électrodes indépendantes. Dans une configuration ACf, le commutateur 566 relie les électrodes 1201 et 1209 (1202 et 1210 pour l'agencement ACf droite) aux bornes de la source de courant alternatif 814 et le commutateur 566 relie les électrodes 1203 et 1207 (1204 et 1208 pour l'agencement ACf droite) aux bornes du voltmètre 562. Cette configuration permet faire un IPG dans le pied. Le commutateur 566 peut désactiver les électrodes 1205 (1206 pour l'agencement ACf droite) en les déconnectant ou en les mettant à la masse.

. Dans une configuration ACb, le commutateur 566 relie une ou deux parmi les électrodes 1201 et 1203 et une ou deux parmi les électrodes 1202 et 1204 aux bornes du voltmètre 562 et relie une ou deux parmi les électrodes 1207 et 1209 et une ou deux parmi les électrodes 1208 ou 1210 aux bornes de la source de tension alternatif 814. Cette configuration ACb permet de faire un IPG en arc-de-jambe ou un BIA entre les jambes.

. Dans le cas d'une configuration ACs (BIA segmentale) avec la station 100 qui inclut la poignée 110, certaines des électrodes du groupe gauche LG ou le groupe droit RG peuvent être connectée à une borne de la source de courant alternatif 814 ou au voltmètre 562 sans que d'autres électrodes du même groupe LG, RG ne soient connectées à l'autre borne à la source de courant alternatif 814 ou au voltmètre 562.

. Dans cet agencement 1200, les électrodes passives 1205, 1206 peuvent ne pas être activées en configuration ACf (pour l'IPG dans le pied). Cela signifie que la distance Δ peut être augmentée par rapport à l'agencement 1100, en plaçant une électrode inactive entre les électrodes reliées à la source de courant alternatif en configuration ACf. Cela permet d'améliorer la qualité des signaux d'impédancemétrie (IPG dans le pied), tout en ayant une surface d'anode et de cathode supérieure par rapport à l'agencement 1100. L'électrode passive (HiZ) peut être plus petite que les autres ; comme il y a quatre autres électrodes indépendantes, les configurations ACf et ACb peuvent toutes être mises en œuvre.

. L'agencement 1200 permet ainsi de faire un BIA entre les jambes, un IPG entre les jambes et un IPG dans le pied.

. Dans une variante de l'agencement 1200, les électrodes 1205 et 1203 (respectivement 1206 et 2014) peuvent être inversées ou bien les électrodes 1205 et 2017 (respectivement 1206 et 2018) peuvent être inversées.

. L'agencement 1200 permet ainsi, sur une base 102, d'effectuer un ESC qui couvre la majeure partie du pied (avec notamment une séparation pied droit pied gauche possible grâce à l'électrode haute impédance), tout en ayant une base 102 fortement polyvalente qui peut effectuer de l'impédancemétrie avec un haut degré de granularité, notamment de l'IPG dans le pied avec une distance Δ entre électrodes augmentée pour avoir une bonne qualité de signal.

. La figure 13 illustre deux agencements 1300a, 1300b qui permettent d'augmenter encore la distance Δ entre les électrodes tout en maintenant une bonne qualité pour l'ESC. Chaque groupe LG, RG comprend six électrodes indépendantes 1301, 1303, 1305, 1307, 1309, 1311, 1313 et 1302, 1304, 1306, 1308, 1310, 1312. Les fonctions des électrodes indiquées sur la figure 9 sont sous la forme « CC/ACf (droite et gauche)/ACb ». Les agencements ACf droite et gauche ne sont pas mis en œuvre simultanément (pour des questions d'acquisition des données par la circuiterie de contrôle et de fonctionnement du commutateur).

. En configuration CC, le commutateur 566 relie les électrodes 1301, 1303, 1307, 1309, 1311, 1313 (LG) et 1302, 1304, 1308, 1310, 1312 (RG) à la source de tension continue 806 (anode/cathode ou cathode/anode). Dans une variante non-illustrée, les électrodes 1305 et 1306 sont elles aussi reliées à la source de tension continue 806 (reliées à la même borne que les autres électrodes de leur groupe d'électrodes) ; dans une variante illustrée, le commutateur 566 relie les électrodes 1305 et 1306 à la haute impédance HiZ. Les électrodes 1305 et 1307 (respectivement 1306 et 1308) peuvent être inversées. On privilégie des électrodes centrales le long de la longueur Y pour les électrodes passives durant l'ESC. La superficie des électrodes est choisie de sorte que la surface des électrodes actives en configuration CC vérifie le critère de surface mentionné précédemment. La configuration avec six électrodes indépendantes permet de vérifier encore plus franchement le critère de surface mentionné précédemment sans compromettre la possibilité d'effectuer une pluralité de mesures différentes (ESC, BIA, IPG, etc.) ni la qualité des mesures. En particulier, si les électrodes 1301 à 2012 ont toute la même surface, le ratio de la surface des électrodes actives sur la surface totale est de plus de 83% (5/6). Si les électrodes 1305, 1306 (électrodes configurées pour être passive en configuration CC) sont plus petites que les autres, le ratio est encore meilleur.

. Comme pour les agencements 1100 et 1200, la configuration ACf est possible car chaque groupe d'électrode LG, RG comprend au moins quatre électrodes indépendantes. Dans une configuration ACf, le commutateur 566 relie les électrodes 1301 et 1311 (respectivement 1302 et 1312) aux bornes de la source de courant alternatif 814 et le commutateur 566 relie les électrodes 1303 et 1309 (1304 et 1310) aux bornes du voltmètre 562. Cette configuration permet faire un IPG dans le pied. Le commutateur 566 peut désactiver les électrodes 1305, 1306, 1307, 1308 en les déconnectant ou en les mettant à la masse.

. Dans une configuration ACb, le commutateur 566 relie une, deux ou trois parmi les électrodes 1301, 1303, 1305 (par exemple deux, comme illustré) et une, deux ou trois parmi les électrodes 1302, 1304, 1306 (par exemple deux, comme illustré) aux bornes du voltmètre 562 et relie une, deux ou trois parmi les électrodes 1307, 1309 et 1311 (par exemple deux, comme illustré) et une, deux ou trois parmi les électrodes 1308, 1310 et 1312 (par exemple deux, comme illustré) aux bornes de la source de courant alternatif 814. Cette configuration ACb permet de faire un IPG en arc-de-jambe ou un BIA entre les jambes.

. En configuration ACs, (BIA segmentale) avec la station 100 qui inclut la poignée 110 certaines des électrodes du groupe gauche LG ou le groupe droit RG peuvent être connectée à la source de courant alternatif 814 ou au voltmètre 562 sans que d'autres électrodes du même groupe ne soit connecté à la source de tension alternative 814 ou au voltmètre 562.

. Dans cet agencement 1300a, les électrodes 1305, 1307 (et 1306, 1308) peuvent ne pas être activées en configuration ACf (pour de l'IPG dans le pied). Cela signifie que la distance Δ peut être augmentée, par rapport à l'agencement 1200, en plaçant deux électrodes inactives entre les électrodes reliées à la source de courant alternatif en configuration ACf. Cela permet d'améliorer la qualité des signaux d'impédancemétrie (IPG dans le pied), tout en ayant un ratio Sa/ST (celui du critère de surface) supérieure par rapport à l'agencement 1200.

. L'agencement 1300a permet ainsi de faire un BIA entre les jambes, un IPG entre les jambes et un IPG dans le pied.

. Dans une variante de l'agencement 1300a, non illustrée, les électrodes 1305 et 1307 (respectivement 1306 et 1308) peuvent être inversées.

. L'agencement 1300b est une variante de l'agencement 1300a dans lequel, pour chacun des groupes RG, LG, l'électrode 1307 a une surface supérieure aux autres, notamment aux électrodes 1303 ou 1309. En particulier, l'électrode 1307 s'étend, le long de la longueur Y, de part et d'autre de l'électrode 1305 (elle forme donc une électrode double). Grâce à l'agencement 1300b, la distance Δ est encore augmentée en plaçant trois chemins conducteurs d'électricité inactifs (une électrode et une électrode double) entre les électrodes reliées à la source de courant alternatif en configuration ACf. Dans cet agencement, l'électrode 1307 est préférablement inactive en configuration AC (ACb notamment). Le ratio Se/Sr peut être supérieur à 100% (ou au moins 90%), grâce à la multiplicité des électrodes le long de la longueur Y de la base 102. Dans un exemple, les bandes de la face supérieure correspondant aux électrodes 1301-1312 ont une dimension selon la longueur Y comprise entre 1,5 cm et 2 cm (par exemple 1,7cm) ; l'espacement entre deux bandes successives peut être compris entre 0,5 cm et 1 cm (par exemple 0,85 cm) ; les électrodes peuvent avoir une dimension selon la largeur X supérieure à 10 cm.

. Les agencements 1300a, 1300b permet ainsi, sur une base 102, d'effectuer un ESC qui couvre la majeure partie du pied (avec notamment une séparation pied droit pied gauche possible grâce à l'électrode haute impédance), tout en ayant une base 102 fortement polyvalente qui peut effectuer de l'impédancemétrie avec un haut degré de granularité, notamment de l'IPG dans le pied avec une distance Δ entre électrodes important pour avoir une meilleure qualité de signal.

. La figure 14 illustre un agencement 1400 qui reprend en commun un certain nombre de caractéristiques de l'agencement 1300b, sauf qu'au lieu de doubler l'électrode 1307, une autre électrode indépendante est ajoutée. Chaque groupe LG, RG comprend ainsi sept électrodes indépendantes 1401, 1403, 1405, 1407, 1409, 1411, 1413 (LG) et 1402, 1404, 1406, 1408, 1410, 1412, 1414 (RG). Les fonctions des électrodes indiquées sur la figure 12 sont sous la forme « CC/ACf (droite et gauche)/ACb ». Les agencements ACf droite et gauche ne sont pas mis en œuvre simultanément (pour des questions d'acquisition des données par la circuiterie de contrôle et de fonctionnement du commutateur).

. En configuration CC, le commutateur 566 relie les électrodes 1401, 1403, 1405, 1409, 1411, 1413, 1413 (LG) et 1402, 1404, 1406, 1410, 1412, 1414 (RG) à la source de tension continue 806 (anode/cathode ou cathode/anode). Dans une variante non illustrée, les électrodes 1407 et 1408 sont elles aussi reliées à la source de tension continue 806 (reliées à la même borne que les autres électrodes de leur groupe d'électrodes LG, RG) ; dans une variante illustrée, le commutateur 566 relie les électrodes 1407 et 1408 à la haute impédance HiZ. L'électrode 1407 peut être inversée avec l'une des électrodes 1405 ou 1409 (respectivement 1406 et 1408). On privilégie des électrodes centrales pour les électrodes passives durant l'ESC. La superficie des électrodes est choisie de sorte que la surface des électrodes actives en configuration CC vérifie le critère de surface mentionné précédemment. La configuration avec sept électrodes indépendantes permet de vérifier encore plus fortement le critère de surface mentionné précédemment sans compromettre la possibilité d'effectuer une pluralité de mesures différentes (ESC, BIA, IPG, etc.) ni la qualité des mesures. En particulier, si les électrodes 1401 à 1414 ont toute la même surface, le ratio de la surface des électrodes actives sur la surface totale est de plus de 85% (six bandes sur sept).

. Comme pour les agencements 1100, 1200, 1300a, 1300b, la configuration ACf est possible car chaque groupe d'électrode LG, RG comprend au moins quatre électrodes indépendantes. Dans une configuration ACf, le commutateur 566 relie les électrodes 1401 et 1413 (respectivement 1402 et 1414) à la source de courant alternatif 814 et le commutateur 566 relie les électrodes 1403 et 1411 (respectivement 1404 et 1412) aux bornes du voltmètre 562. Le commutateur 566 peut désactiver les électrodes 1405, 1406, 1407, 1408, 1409, 1410 en les déconnectant ou en les mettant à la masse. Cette configuration permet faire un IPG dans le pied.

. Dans une configuration ACb, le commutateur 566 relie une, deux ou trois électrodes parmi les électrodes 1401, 1403, 1405 (par exemple les trois, comme illustré) et une, deux ou trois parmi les électrodes 1402, 1404, 1406 (par exemple les trois, comme illustré) aux bornes du voltmètre 562 et relie une, deux ou trois parmi les électrodes 1409, 1411, 1413 (par exemple deux, comme illustré) et une, deux ou trois électrodes parmi les électrodes 1410, 1412, 1414 (par exemple deux, comme illustré) aux bornes de la source de courant alternatif 814. Cette configuration ACb permet de faire un IPG en arc-de-jambe ou un BIA entre les jambes.

. En configuration ACs (BIA segmentale) avec la station 100 qui inclut la poignée 110 certaines des électrodes du groupe gauche LG ou le groupe droit RG peuvent être connectée à la source de courant alternatif 814 ou au voltmètre 566 sans que d'autres électrodes du même groupe ne soit connecté à la source de courant alternatif 814 ou au voltmètre 566.

. Dans cet agencement 1400, les électrodes 1405, 1407, 1409 peuvent ne pas être activées en configuration AC pour de l'IPG dans le pied. Cela signifie que la distance Δ est augmentée, par rapport à l'agencement 1200, en plaçant trois électrodes inactives entre les électrodes reliées à la source de courant alternatif en configuration ACf. Cela permet améliorer la qualité des signaux d'impédancemétrie (IPG dans le pied).

. L'agencement 1400 permet ainsi de faire un BIA entre les jambes, un IPG entre les jambes et un IPG dans le pied.

. Dans une variante de l'agencement 1400, les électrodes 1405 et 1407 (respectivement 2206 et 2208) peuvent être inversées.

. L'agencement 1400 permet ainsi, sur une base 102, d'effectuer un ESC qui couvre la majeure partie du pied (avec notamment une séparation pied droit pied gauche possible grâce à l'électrode haute impédance), tout en ayant une base 102 fortement polyvalent qui peut effectuer de l'impédancemétrie avec un haut degré de granularité, notamment de l'IPG dans le pied avec une distance Δ entre électrodes optimisée pour avoir une bonne qualité de signal. La sensibilité à la position du pied en largeur X et en longueur Y est assez faible, ce qui assure une bonne répétabilité des mesures.

. Dans cet agencement, trois électrodes sont inactives en configuration ACf. Cela permet de plus d'avoir des électrodes pour le BIA ou l'IPG qui aient une surface réduite, ce qui améliore la qualité des signaux. En effet, l'injection de courant et la prise de tension peuvent être meilleures lorsqu'elles sont localisées sur le pied (par « localisé », il est signifié sur une surface réduite sous le pied). Inversement, ces trois électrodes inactives en configuration ACf deviennent deux électrodes actives et une passive en configuration CC, de sorte que la surface des électrodes est dans cette configuration maximisée. Cette configuration à sept électrodes indépendantes offre une surface d'électrode active importante en configuration AC tout en permettant de multiplier les types de mesures sur les pieds ou sur chaque pied avec une bonne qualité de signal.

. Dans toutes les configurations AC présentés dans la description, les connexions aux bornes de la source de courant alternatif 814 peuvent être inversées (i+ devient i- et i- devient i+) ; de la même façon, les connexions aux bornes du voltmètre 562 peuvent être inversées (V+ devient V- et V- devient V+).

. Dans toutes les configurations AC présentées dans la description, la connexion entre la source de courant alternatif 814 et le voltmètre 562 peut être inversé (V devient i et i devient V). Pour les configurations ACf, il est toutefois préférable d'avoir les électrodes V+ et V- à l'intérieur du segment du corps d'un utilisateur parcouru par le courant généré entre les électrodes i+ et i+. Dans le cas inverse, on ne contrôle alors pas le passage du courant pour les portions de segments entre les électrodes i et les électrodes V.

. Dans les agencements présentés précédemment certaines électrodes peuvent assurer au moins deux, trois ou quatre fonctions différentes. La division en une pluralité de bandes le long de la longueur Y disposées parallèlement le long de la largeur X permet une modularité augmentée tout en maintenant de bonnes qualités de mesure.

. Dans un mode de réalisation, la station de mesure 100 permet de prendre un ECG. L'ECG peut être pris entre les jambes, entre les mains (électrodes RA, LA sur la figure 4) ou entre les mains (électrodes RA, LA) et les jambes. Une des électrodes du groupe gauche LG peut alors être connecté par le commutateur 566 en électrode LL (« *left leg* »). Une des électrodes du groupe droite RG peut alors être connectée par le commutateur 566 en électrode RG. Les électrodes non connectées à la source de courant continu 814 ou au voltmètre 562 sont inactives (par exemple déconnectées).

. Dans tous les agencements décrits, la dimension en Y (i.e. le long de la direction Y, c'est-à-dire la longueur de la base 102) des électrodes qui sont actives en configuration CC peut être égale ou supérieure à la dimension en Y électrodes qui sont passives en configuration CC. Cela permet de maximiser la surface du pied en contact avec les électrodes actives. Les agencements 1000 et 1200 illustre un cas où l'électrode passive 1003, 1004, 1205, 1206 est plus petite le long de la longueur Y que les électrode actives 1001, 1005, 1002, 1006, 1203, 1207, 1204, 1208. Cet agencement est rendu possible du fait de la multiplicité des électrodes qui permet de dédier une électrode à la haute impédance tout en ayant suffisamment d'électrodes, de taille adaptée, pour effectuer les autres mesures. Les agencements 1300, 1400 illustrent des cas où l'électrode passive 1305, 1306, 1407, 1408 présente une même taille le long de la dimension Y que les électrodes actives. En particulier, plus il y a un nombre élevé d'électrodes indépendantes par groupe LG, RG, plus la surface de chaque électrode est faible. Dès lors, connecter une seule électrode à la haute impédance ne réduit pas significativement la surface de contact entre la peau et l'anode ou la cathode.

. Dans les agencements à trois ou plus électrodes indépendantes par groupe d'électrodes RG, LG (agencements 1000, 1100, 1200, 1300a, 1300b, 1400) l'électrode qui est passive en configuration CC est positionnée, le long de la longueur Y, entre deux électrodes qui sont actives en configuration CC. Ce positionnement présente deux avantages : il augmente la probabilité que l'utilisateur touche l'électrode passive (un décalage du pied le long de la longueur Y de la base 102 n'aura pas de conséquence) et il permet de limiter la surface de contact perdue avec le pied pour l'ESC : du fait de la courbure du pied, le contact entre l'électrode positionnée sous l'arche de la voûte plantaire est plus faible que pour les autres électrodes. Par conséquent, la perte de surface pour l'ESC est plus faible que pour une autre électrode.

. Alternativement, l'électrode passive en configuration CC peut être placée comme une électrode extrémale. Cela permet de profiter du contact avec le talon ou la pointe des pieds pour s'assurer que le corps d'un utilisateur est bien mis à la haute impédance.

. Dans tous les agencements décrits, les électrodes qui sont en position extrémale le long de la longueur Y peuvent présenter une surface plus importante que les autres électrodes. Un tel agencement 1500 est illustré notamment sur la figure 15, sur laquelle les électrodes extrémales (1501, 1502, 1508, 1509) peuvent avoir une dimension le long de la longueur Y plus importante (par exemple deux fois plus importante) que les autres électrodes (1503, 1504, 1506, 1507). Lorsque les électrodes ont la même forme, les deux formulations sont équivalentes.

. Cela signifie que sur les agencements 1000, 1100, 1200, 1300a, 1300b, 1400, les électrodes extrémales le long de la longueur Y peuvent avoir une dimension le long de la longueur Y plus importante que celle des chemins conducteurs d'électricité compris entre les deux chemins conducteurs d'électricité extrémaux. Par exemple, sur la figure 6, les couples de bandes L1 et L3, L15 et L17, R2 et R4, R16 et R18 ne sont pas indépendants électriquement mais sont reliés en permanence au niveau du circuit électrique. Cette configuration permet d'augmenter la taille des électrodes extrémales sans perturber l'aspect visuel de la face supérieure 604.

. Les électrodes peuvent avoir une dimension constante le long de largeur X de la base 102. Cela permet de limiter la sensibilité des mesures en fonction de la position du pied le long de la largeur X. Cela signifie que le long de la largeur X, les électrodes d'un même groupe RG, LG commencent et se terminent au même endroit. Formulé autrement, toute droite parallèle à la longueur Y passant par un chemin de conduction électrique d'un groupe passe par tous les chemins conducteurs d'électricité du groupe. Dans un mode de réalisation, sur la face supérieure 604, les électrodes ont chacune une forme rectangulaire, avec une dimension le long de la largeur X supérieure à la dimension le long de la longueur Y (rapport de cinq au moins). Sur la face supérieure 604, les rectangles peuvent avoir les mêmes dimensions.

. Dans un mode de réalisation, les électrodes s'étendent le long de la largeur X de la station de mesure 100 sur au moins 40%, voire 45%, de la largeur X100 de la station de mesure. On s'assurer ainsi que le pied touche bien les électrodes, quel que soit son placement le long de la largeur X.

. Le fait d'avoir un agencement régulier des bandes sur la face supérieure peut inciter l'utilisateur à se positionner entre les bandes extrémales et donc permet que l'utilisateur se place naturellement de façon correcte. De plus, en ayant des électrodes extrémales plus grande, la probabilité que le pied touche les deux électrodes extrémales est augmentée.

. Un agencement en bandes identiques permet d'avoir un aspect régulier sur la face supérieure, ce qui contribue à supprimer l'effet « médical » de la station de mesure 100 et ainsi à renforcer la rétention du produit (utilisation de la station dans le temps). En d'autres termes, l'utilisateur a l'impression d'utiliser un produit de confort et non pas un produit qui lui permet de surveiller des paramètres physiologiques.

. La distance Δ peut être d'au moins 8 cm, voire au moins 9 cm. En particulier, il existe une contrainte relative à la longueur minimale d'un pied : si la distance Δ est trop importante, un pied de petite taille ne pourra pas être en contact avec les quatre électrodes du groupe d'électrode RG, LG (pour une configuration ACf).

. Entre deux ESC successifs, le commutateur 566 ou le générateur de tension 806 peut inverser l'anode et la cathode pour permettre de régénérer les électrodes. En effet, une oxydoréduction a lieu à chaque ESC, ce qui dégrade les dépôts métalliques ou les inserts métalliques.

. Dans un mode de réalisation, la station de mesure 100 permet de prendre des ECG de l'utilisateur. Les capteurs 104 utilisés à cette fin sont présents sur le substrat et sur la poignée, pour qu'au moins un pied et une main soient au contact d'une électrode. En particulier, les deux pieds et les deux mains sont en contact avec au moins une électrode.

. Le document WO2021/164561 décrit une balance avec poignée permettant de faire un ECG.

. Pour obtenir une ECG multi voies, le membre inférieur gauche doit être relié à une électrode dite LL (left leg). Grâce au commutateur 566, un agencement ECG peut comprendre au moins une électrode du groupe gauche LG qui est reliée au circuit électrique ECG 568 (par exemple l'électrode L15/L17). Un autre agencement comprend en plus une configuration ACf dans le pied droit, afin de faire un ECG en même temps qu'un IPG dans le pied droit. Alternativement, un autre agencement comprend en plus une configuration ACb, sauf que seule l'électrode L13 parmi les électrodes L13, L15/L17 est connectée au générateur de courant alternatif (l'électrode L15/L17 étant reliée l'électrode LL pour l'ECG).

. La figure 16 représente un schéma électrique avec la logique du commutateur 566 (avec une poignée représentée de façon simplifiée). Le commutateur 566 comprend une pluralité d'interrupteurs, qui sont positionnés par exemple sur le PCB de la base 102 et sur le PCB de la poignée 110. Les références sur la figure 16 y sont semblables à celles précédemment utilisés (Sudo_HiZ étant HiZ, Sudo étant Cath ou An, I étant i, et open signifiant que l'interrupteur est ouvert). Chaque électrodes L1 à R18, LH1, LH2, RH1, RH2 est connectée à la circuiterie de contrôle 550 via un ou plusieurs interrupteurs 1602. Chaque interrupteur 1602 peut être piloté par un ou plusieurs ports GPIO (« *General Purpose Input*/*Output* »), représenté par les valeurs 0 et 1 à côté des connexions des interrupteurs. Un interrupteur à deux positions est piloté par un seul GPIO et un interrupteur à quatre positions est pilotés par deux GPIOs. Les instructions GPIO sont par exemple envoyées par le MCU. En particulier, le commutateur 566 comprend au moins un interrupteur par électrode. Dans les configurations à N électrodes indépendantes (pour un groupe d'électrode), la station de mesure 100 comprend au moins N interrupteurs.

. La figure 17 représente la poignée 110 plus en détail (à l'exception du carré en pointillé qui représente les composants dans la base 102). Le commutateur comprend ici deux interrupteurs-maitre 1702a (dans la poignée), 1702b (dans la base) en vis-à-vis sont des interrupteurs qui comprennent par exemple quatre interrupteurs qui commutent simultanément dans la même position. Ils permettent de commuter entre une configuration ECG et une configuration impédancemétrie (BIA, IPG) en limitant le nombre de fils dans le câble 302 (pour garder une section de câble faible et souple). La poignée 110 comprend au moins un circuit électrique ECG de poignée 1706 relié à au moins une électrode LH1, LH2, RH1, RH2 et configuré pour traiter le signal avant qu'il ne transite par le câble 302 (typiquement un circuit suiveur avec un amplificateur opérationnel). La poignée 110 comprend au moins un circuit électrique d'impédancemétrie de poignée 1706 relié à au moins une électrode LH1, RH1 configuré pour traiter le signal avant qu'il ne transite par le câble 302 (typiquement un circuit suiveur avec un amplificateur opérationnel), pour ensuite aller vers le voltmètre. Les circuits électriques ECG et d'impédancemétrie sont reliés à l'interrupteur-maitre 1702a. LA_DETECT et RA_DETECT font partie d'un système de détection de préhension de la poignée 110 qui n'est pas décrit plus en détail ici. Les autres interrupteurs de la poignée 110 permettent de connecter les électrodes RH1, RH2, LH1, LH2 au circuit électrique ECG ou au circuit électrique BIA. En particulier, le commutateur 566 comprend un interrupteur 1708 qui permet de sélectivement relier l'électrode RH2 au le circuit électrique ECG 1704 ou au circuit d'impédancemétrie 1706 (connecté au voltmètre) ; le commutateur 566 comprend un interrupteur 1710 qui permet de relier l'électrode LH2 au circuit électrique ECG 1704 ou au circuit électrique d'impédancemétrie 1706 (connecté au voltmètre) ; le commutateur 566 comprend un interrupteur 1712 permet de sélectivement relier l'électrodes RH1 à l'électrode RH2 ou à un circuit d'impédancemétrie (connecté à la source de courant) ; le commutateur 566 comprend un interrupteur 1714 permet de sélectivement relier l'électrodes LH1 à l'électrode LH2 ou à un circuit électrique d'impédancemétrie (connecté à la source de courant). Les figures 16 et 17 décrivent donc entièrement la configuration électronique du commutateur 566 permettant de mettre en œuvre les configurations décrites dans la description. Le double interrupteur 1702a, 1702b permet de limiter le nombre de conducteurs passant dans le câble 302. En particulier, la poignée comprend un interrupteur-maitre 1702 et chaque électrode de la poignée est connectée à un interrupteur pour basculer entre un circuit de traitement ECG et un circuit d'impédancemétrie.

. La figure 18 illustre schématiquement un ensemble de configurations pour les électrodes de la station de mesure 100. La poignée 110 et ses quatre électrodes sont représentées ; la base et ses groupes gauche et droite LG, RG, avec leurs sept électrodes chacun, sont représentés. En reprenant les chemins de conductions électriques 602 de la figure 6, les sept électrodes de l'ensemble gauche LG sont : L1 et L3 (qui sont liées), L5, L7, L9, L11, L13, L15 et L17 (qui sont liées) ; et les sept électrodes de l'ensemble droite RG sont : R2 et R4 (qui sont liées), R6, R8, R10, R12, R14, R16 et R18 (qui sont liées). Sur la figure 18, treize configurations pour la fonction des électrodes ont été représentées. Le commutateur de la figure 16 permet d'alterner entre les treize configurations. Chaque tableau comprend quatorze cases (treize fonctions activées et une déconnection totale), qui représente la fonction de l'électrode pour une configuration :
.

| BIA ou IPG (entre les jambes) | BIA / ICG (moitié droite - jambe et bras) | BIA (bras droit) | BIA (jambe gauche) | BIA /ICG (moitié gauche - jambe et bras) | BIA (bras gauche) | BIA (jambe gauche) |
|---|---|---|---|---|---|---|
| IPG (dans le pied droit) | IPG (dans le pied gauche) | ECG avec IPG (dans le pied droit) | ECG avec IPG (entre les jambes) | ESC (anode à gauche) | ESC (anode à droite) | Déconnexion |

. Sur la figure 18, i+ et i- sont les électrodes reliées à la source de courant alternatif (configuration AC) ; V+ et V- sont les électrodes reliées au voltmètre (configuration AC) ; LL, LA, RA sont les électrodes reliées au système d'acquisition d'ECG (configuration ECG) ; A et K sont les électrodes reliées à la source de tension continue (configuration CC) ; Z sont les électrodes reliées à la haute impédance (configuration CC).

. Pour chacune des configurations de la figure 17, une combinaison des ports GPIO est représentée sur la figure 16 qui permet la connexion appropriée des électrodes.

. Le commutateur 566 peut aussi déconnecter l'intégralité des électrodes de la base 102.

. Dans un mode de réalisation, les électrodes sont toutes en ITO (indium-tin-oxyde). L'usage de ce matériau est documenté pour l'impédancemétrie. Néanmoins, pour l'ESC, le matériau utilisé comprend généralement de l'acier ou en nickel. Les inventeurs se sont aperçus que le ITO offrait des performances équivalentes tout en ayant des propriétés de dépôt supérieures.

. La station de mesure 100 permet d'effectuer une pluralité de mesures différentes (ESC, BIA, IPG, etc.) en attribuant différentes fonctions aux mêmes chemins conducteurs d'électricité. Avec le commutateur 566 permet d'alterner entre les configurations. La mémoire peut stocker un programme qui comprend des instructions qui, une fois exécutée, par le processeur, permet de mettre en œuvre différentes méthodes.

. Selon un mode de réalisation, la circuiterie de contrôle 550 met en œuvre la méthode suivante : (E1) activation du commutateur 556 pour mettre les électrodes dans une configuration, (E2) déclenchement d'une mesure associée à la configuration, (E3) activation du commutateur 556 pour mettre les électrodes dans une autre configuration, (E4) déclenchement d'une mesure associée à l'autre configuration. Le changement de configuration peut notamment être n'importe laquelle des configurations décrites dans la description et notamment en figure 18 : CC vers AC, dont CC vers ACf ou CC vers ACb. CC vers ACf peut être CC vers ACf pied gauche ou CC vers ACf pied droit, ACf vers ACb, ACb vers une autre ACb, etc.

## Revendications

1. Station de mesure comprenant :
un groupe gauche (LG) d'électrodes, comprenant au moins deux électrodes électriquement indépendantes agencées pour être au contact du dessous d'un pied gauche d'un utilisateur,
un groupe droite (RG) d'électrodes, comprenant au moins deux électrodes électriquement indépendantes agencées pour être au contact du dessous d'un pied droit de l'utilisateur,
une source de tension continue (806),
un commutateur (566) configuré pour activer et désactiver une configuration dite à courant continu, dite configuration CC, dans laquelle au moins une électrode, dite électrode active, d'au moins un groupe d'électrodes (LG, RG) est connectée à la source de tension continue (806),
une source de courant alternatif (814) ;
dans laquelle le commutateur (566) est configuré pour sélectivement activer ou désactiver une configuration dite à courant alternatif, dite configuration AC (ACs, ACf, ACb), dans laquelle au moins une électrode parmi les électrodes du groupe gauche d'électrodes et du groupe droite d'électrodes est connectée à la source de courant alternatif, et
dans laquelle, pour un groupe (LG, RG), la surface de la ou des électrodes qui sont connectées à la source de tension continue (806) en configuration CC représente plus de 50% de la surface des électrodes du groupe (LG, RG), voire plus de 75%.

2. Station de mesure selon la revendication 1, dans laquelle en configuration CC, au moins une électrode, dite électrode passive, d'au moins un groupe d'électrodes n'est pas reliée à la source de tension continue (806).

3. Station de mesure selon la revendication 1 ou 2, dans laquelle, dans une configuration AC (ACf, ACb), le commutateur (566) est configuré pour connecter :
au moins deux électrodes parmi les électrodes du groupe gauche d'électrodes (LG) et du groupe droit d'électrodes (RG) aux bornes de la source de courant alternatif (814), par exemple :
- au moins une électrode du groupe gauche (RG) et au moins une électrode du groupe droite (RD) aux bornes de la source de courant alternatif (814), ou
- au moins une électrode d'un groupe à une borne de la source de courant et une autre électrode du même groupe à une autre borne de la source de courant.

4. Station de mesure selon l'une quelconque des revendications 1 à 3, dans laquelle :
le groupe gauche (LG) et/ou le groupe droite (RG) comprend chacun trois électrodes électriquement indépendantes (1001, 1002, 1003, 1004, 1005, 1006),
en configuration CC, le commutateur (566) est configuré pour connecter au moins deux électrodes (1001, 1005) du groupe gauche (LG) et/ou au moins deux électrodes (1002, 1006) du groupe droite (RG) à la source de tension continue (806),
ou
le groupe gauche (LG) et/ou le groupe droite (RG) comprend chacun quatre électrodes électriquement indépendantes, et
en configuration CC, le commutateur (566) est configuré pour connecter au moins trois électrodes du groupe gauche et/ou au moins trois électrodes du groupe droite à la source de tension continue (806),
ou
le groupe gauche (LG) et/ou le groupe droite (RG) comprend chacun cinq électrodes électriquement indépendantes,
en configuration CC, le commutateur est configuré pour relier au moins quatre électrodes du groupe gauche (LG) et/ou au moins quatre électrodes du groupe droite (RG) à la source de tension continue (806).

5. Station de mesure selon les revendications 2 et 4, dans laquelle, en configuration CC, l'électrode passive (1003, 1004, 1103, 1104, 1205, 1207, 1305, 1306, 1407, 1408, ...) est située entre deux électrodes actives.

6. Station de mesure selon l'une quelconque des revendications 1, 3 ou 4, dans laquelle, en configuration CC, toutes les électrodes du groupe gauche (LG) et du groupe droite (RG) sont reliées à la source de tension continue (806).

7. Station de mesure selon l'une quelconque des revendications 1 à 6, dans laquelle, en configuration CC, un groupe d'électrodes fonctionne en cathode et un groupe d'électrodes fonctionne en anode.

8. Station de mesure selon l'une quelconque des revendications 1 à 7, comprenant en outre une unité de contrôle (810), apte à contrôler la source de tension continue (806) et à contrôler un circuit de mesure (812) permettant notamment à l'unité de contrôle (810) de mesurer le courant traversant les pieds de l'utilisateur.

9. Station selon l'une quelconque des revendications 1 à 8, dans laquelle
les électrodes sont espacées les unes des autres, dans le sens d'une longueur de la station de mesure, les pieds de l'utilisateur se positionnant en utilisation normale le long de la longueur de la station de mesure,
et/ou
les électrodes extrémales le long de la longueur sont plus larges.

10. Station de mesure selon l'une quelconque des revendications 1 à 9, dans lequel, pour un groupe d'électrodes, les électrodes configurées pour être actives en configuration CC couvrent en surface (Sa) au moins 50% de la surface (Se) de l'enveloppe convexe définie par toutes les électrodes du groupe, et/ou la distance (Dmax) entre les électrodes extrémales, électrodes incluses, le long d'une longueur de la station de mesure est d'au moins 20 cm, les pieds de l'utilisateur se positionnant en utilisation normale le long de la longueur de la station de mesure.

11. Station de mesure selon l'une quelconque des revendications 1 à 10, dans laquelle les électrodes du groupe gauche s'étendent le long d'une largeur de la station de mesure (100) sur plus de 40% de la largeur de la station de mesure et les électrodes du groupe droite s'étendent le long d'une largeur de la station de mesure sur plus de 40% de la largeur de la station de mesure.

12. Station de mesure selon l'une quelconque des revendications 1 à 11, dans lequel les électrodes des groupes gauche et droite comprennent un matériau en oxyde d'indium-étain, ITO.

13. Station de mesure selon l'une quelconque des revendications 1 à 12, dans laquelle la source de tension continue est configurée pour sélectivement appliquer à une paire d'électrodes, dites actives, des paliers successifs de tension constante, lesdites électrodes de la paire constituant une anode et une cathode, dans laquelle, par exemple, les valeurs de tension des paliers successifs sont décroissantes et/ou durent entre 500ms et 2s chacun.

14. Station de mesure selon l'une quelconque des revendications 1 à 13, comprenant en outre un capteur de poids.

15. Station de mesure selon l'une quelconque des revendications 1, ou 3 à 14, comprenant :
- une base (102) comprenant le groupe gauche d'électrode (LG) et le groupe droit d'électrode (RG),
- une poignée (110) avec au moins une électrode apte à être au contact de la main,
dans laquelle, en configuration CC, le commutateur (566) est configuré pour connecter toutes les électrodes de la base (102) aux bornes de la source de tension continue (806) et pour connecter l'électrode de la poignée (110) à une haute impédance (HiZ), supérieure à 500 kOhm.

## Patentansprüche

1. Messstation, umfassend:
eine linke Elektrodengruppe (LG), die mindestens zwei elektrisch voneinander unabhängige Elektroden umfasst, die so angeordnet sind, dass sie die Unterseite des linken Fußes eines Benutzers berühren,
eine rechte Elektrodengruppe (RG), die mindestens zwei elektrisch unabhängige Elektroden umfasst, die so angeordnet sind, dass sie die Unterseite des rechten Fußes des Benutzers berühren,
eine Gleichspannungsquelle (806),
einen Schalter (566), der so konfiguriert ist, dass er eine sogenannte Gleichstromkonfiguration, auch CC-Konfiguration genannt, aktiviert und deaktiviert, in der mindestens eine Elektrode, die sogenannte aktive Elektrode, mindestens einer Elektrodengruppe (LG, RG) mit der Gleichspannungsquelle (806) verbunden ist,
eine Wechselstromquelle (814),
wobei der Schalter (566) so konfiguriert ist, dass er selektiv eine sogenannte Wechselstromkonfiguration, auch als AC-Konfiguration (ACs, ACf, ACb) bezeichnet, aktiviert oder deaktiviert, in der mindestens eine Elektrode aus den Elektroden der linken Elektrodengruppe und der rechten Elektrodengruppe mit der Wechselstromquelle verbunden ist, und
wobei für eine Gruppe (LG, RG) die Fläche der Elektrode(n), die in der Gleichstromkonfiguration (CC) mit der Gleichspannungsquelle (806) verbunden ist (sind), mehr als 50 % der Fläche der Elektroden der Gruppe (LG, RG) oder sogar mehr als 75 % beträgt.

2. Messstation nach Anspruch 1, wobei in der Gleichstromkonfiguration mindestens eine Elektrode, die als passive Elektrode bezeichnet wird, aus mindestens einer Elektrodengruppe nicht mit der Gleichspannungsquelle (806) verbunden ist.

3. Messstation nach Anspruch 1 oder 2, wobei in einer Wechselstromkonfiguration (ACf, ACb) der Schalter (566) so konfiguriert ist, dass er:
mindestens zwei Elektroden aus der linken Elektrodengruppe (LG) und der rechten Elektrodengruppe (RG) an die Anschlüsse der Wechselstromquelle (814) anzuschließen, beispielsweise:
- mindestens eine Elektrode der linken Gruppe (RG) und mindestens eine Elektrode der rechten Gruppe (RD) an die Anschlüsse der Wechselstromquelle (814) anzuschließen, oder
- mindestens eine Elektrode einer Gruppe an einen Anschluss der Stromquelle und eine weitere Elektrode derselben Gruppe an einen anderen Anschluss der Stromquelle.

4. Messstation nach einem der Ansprüche 1 bis 3, wobei:
die linke Gruppe (LG) und/oder die rechte Gruppe (RG) jeweils drei elektrisch unabhängige Elektroden (1001, 1002, 1003, 1004, 1005, 1006) umfasst,
in einer Gleichstromkonfiguration der Schalter (566) so konfiguriert ist, dass er mindestens zwei Elektroden (1001, 1005) der linken Gruppe (LG) und/oder mindestens zwei Elektroden (1002, 1006) der rechten Gruppe (RG) mit der Gleichspannungsquelle (806) verbindet,
oder
die linke Gruppe (LG) und/oder die rechte Gruppe (RG) jeweils vier elektrisch unabhängige Elektroden umfasst, und
in der Gleichstromkonfiguration ist der Schalter (566) so konfiguriert, dass er mindestens drei Elektroden der linken Gruppe und/oder mindestens drei Elektroden der rechten Gruppe mit der Gleichspannungsquelle (806) verbindet,
oder
die linke Gruppe (LG) und/oder die rechte Gruppe (RG) jeweils fünf elektrisch unabhängige Elektroden umfasst,
in der Gleichstromkonfiguration der Schalter so konfiguriert ist, dass er mindestens vier Elektroden der linken Gruppe (LG) und/oder mindestens vier Elektroden der rechten Gruppe (RG) mit der Gleichspannungsquelle (806) verbindet.

5. Messstation nach den Ansprüchen 2 und 4, wobei in der DC-Konfiguration die passive Elektrode (1003, 1004, 1103, 1104, 1205, 1207, 1305, 1306, 1407, 1408, ...) zwischen zwei aktiven Elektroden angeordnet ist.

6. Messstation nach einem der Ansprüche 1, 3 oder 4, wobei in der Gleichstromkonfiguration alle Elektroden der linken Gruppe (LG) und der rechten Gruppe (RG) mit der Gleichspannungsquelle (806) verbunden sind.

7. Messstation nach einem der Ansprüche 1 bis 6, bei der in einer Gleichstromkonfiguration eine Elektrodengruppe als Kathode und eine Elektrodengruppe als Anode fungiert.

8. Messstation nach einem der Ansprüche 1 bis 7, die ferner eine Steuereinheit (810) umfasst, die in der Lage ist, die Gleichspannungsquelle (806) zu steuern und eine Messschaltung (812) zu steuern, die es der Steuereinheit (810) insbesondere ermöglicht, den durch die Füße des Benutzers fließenden Strom zu messen.

9. Station nach einem der Ansprüche 1 bis 8, wobei
die Elektroden in Längsrichtung der Messstation voneinander beabstandet sind, wobei sich die Füße des Benutzers im Normalbetrieb entlang der Längsachse der Messstation positionieren,
und/oder
die Endelektroden entlang der Länge breiter sind.

10. Messstation nach einem der Ansprüche 1 bis 9, wobei bei einer Elektrodengruppe die Elektroden, die so konfiguriert sind, dass sie im Gleichstrommodus aktiv sind, flächenmäßig (Sa) mindestens 50 % der Fläche (Se) der durch alle Elektroden der Gruppe definierten konvexen Hülle abdecken, und/oder der Abstand (Dmax) zwischen den Endelektroden, einschließlich dieser, entlang einer Längsachse der Messstation beträgt mindestens 20 cm, wobei sich die Füße des Benutzers bei normaler Nutzung entlang der Längsachse der Messstation positionieren.

11. Messstation nach einem der Ansprüche 1 bis 10, wobei sich die Elektroden der linken Gruppe entlang einer Breite der Messstation (100) über mehr als 40 % der Breite der Messstation erstrecken und sich die Elektroden der rechten Gruppe entlang einer Breite der Messstation über mehr als 40 % der Breite der Messstation erstrecken.

12. Messstation nach einem der Ansprüche 1 bis 11, wobei die Elektroden der linken und rechten Gruppe ein Indium-Zinn-Oxid-Material (ITO) umfassen.

13. Messstation nach einem der Ansprüche 1 bis 12, wobei die Gleichspannungsquelle so ausgelegt ist, dass sie an ein Paar sogenannter aktiver Elektroden selektiv aufeinanderfolgende Stufen konstanter Spannung anlegt, wobei die Elektroden des Paares eine Anode und eine Kathode bilden, wobei beispielsweise die Spannungswerte der aufeinanderfolgenden Stufen abnehmen und/oder jeweils zwischen 500 ms und 2 s andauern.

14. Messstation nach einem der Ansprüche 1 bis 13, die ferner einen Gewichtssensor umfasst.

15. Messstation nach einem der Ansprüche 1 oder 3 bis 14, umfassend:
- einen Sockel (102), der die linke Elektrodengruppe (LG) und die rechte Elektrodengruppe (RG) umfasst,
- einen Griff (110) mit mindestens einer Elektrode, die mit der Hand in Kontakt kommen kann,
wobei in der Gleichstromkonfiguration der Schalter (566) so ausgelegt ist, dass er alle Elektroden der Basis (102) an die Anschlüsse der Gleichspannungsquelle (806) anschließt und die Elektrode des Griffs (110) an eine hohe Impedanz (HiZ) von mehr als 500 kOhm anschließt.

## Claims

1. A measurement station comprising:
a left group (LG) of electrodes, comprising at least two electrically independent electrodes arranged to be in contact with the underside of a user's left foot,
a right group (RG) of electrodes, comprising at least two electrically independent electrodes arranged to be in contact with the underside of the user's right foot,
a direct current source (806),
a switch (566) configured to activate and deactivate a so-called direct current configuration, referred to as the DC configuration, in which at least one electrode, referred to as an active electrode, of at least one group of electrodes (LG, RG) is connected to the DC voltage source (806),
an alternating current source (814),
wherein the switch (566) is configured to selectively activate or deactivate a so-called alternating current configuration, referred to as the AC configuration (ACs, ACf, ACb), in which at least one electrode from among the electrodes of the left group of electrodes and the right group of electrodes is connected to the alternating current source, and
wherein, for a group (LG, RG), the surface area of the electrode(s) connected to the DC voltage source (806) in DC mode represents more than 50% of the surface area of the electrodes in the group (LG, RG), or even more than 75%.

2. A measuring station according to claim 1, wherein, in DC configuration, at least one electrode, referred to as a passive electrode, of at least one group of electrodes is not connected to the DC voltage source (806).

3. A measuring station according to claim 1 or 2, wherein, in an AC configuration (ACf, ACb), the switch (566) is configured to connect:
at least two electrodes from among the electrodes of the left electrode group (LG) and the right electrode group (RG) to the terminals of the AC power source (814), for example:
- at least one electrode from the left group (RG) and at least one electrode from the right group (RD) to the terminals of the AC power source (814), or
- at least one electrode from one group to one terminal of the current source and another electrode from the same group to another terminal of the current source.

4. A measuring station according to any one of claims 1 to 3, wherein:
the left group (LG) and/or the right group (RG) each comprises three electrically independent electrodes (1001, 1002, 1003, 1004, 1005, 1006),
in DC configuration, the switch (566) is configured to connect at least two electrodes (1001, 1005) of the left group (LG) and/or at least two electrodes (1002, 1006) of the right group (RG) to the DC voltage source (806),
or
the left group (LG) and/or the right group (RG) each comprises four electrically independent electrodes, and
in DC mode, the switch (566) is configured to connect at least three electrodes of the left group and/or at least three electrodes of the right group to the DC voltage source (806),
or
the left group (LG) and/or the right group (RG) each comprises five electrically independent electrodes,
in DC mode, the switch is configured to connect at least four electrodes of the left group (LG) and/or at least four electrodes of the right group (RG) to the DC voltage source (806).

5. A measurement station according to claims 2 and 4, wherein, in DC configuration, the passive electrode (1003, 1004, 1103, 1104, 1205, 1207, 1305, 1306, 1407, 1408, ...) is situated between two active electrodes.

6. A measuring station according to any one of claims 1, 3 or 4, wherein, in DC configuration, all the electrodes of the left group (LG) and the right group (RG) are connected to the DC voltage source (806).

7. A measuring station according to any one of claims 1 to 6, in which, in DC configuration, one group of electrodes operates as a cathode and one group of electrodes operates as an anode.

8. A measurement station according to any one of claims 1 to 7, further comprising a control unit (810), capable of controlling the DC voltage source (806) and of controlling a measurement circuit (812) enabling, in particular, the control unit (810) to measure the current flowing through the user's feet.

9. A station according to any one of claims 1 to 8, in which
the electrodes are spaced apart from one another along the length of the measuring station, the user's feet being positioned, in normal use, along the length of the measuring station,
and/or
the end electrodes along the length are wider.

10. A measurement station according to any one of claims 1 to 9, wherein, for a group of electrodes, the electrodes configured to be active in DC mode cover, in terms of surface area (Sa), at least 50% of the surface area (Se) of the convex envelope defined by all the electrodes of the group, and/or the distance (Dmax) between the end electrodes, including the electrodes themselves, along a length of the measuring station is at least 20 cm, the user's feet being positioned during normal use along the length of the measuring station.

11. A measurement station according to any one of claims 1 to 10, in which the electrodes of the left group extend along a width of the measuring station (100) over more than 40% of the width of the measuring station and the electrodes of the right group extend along a width of the measuring station over more than 40% of the width of the measuring station.

12. A measuring station according to any one of claims 1 to 11, wherein the electrodes of the left and right groups comprise an indium tin oxide (ITO) material.

13. A measuring station according to any one of claims 1 to 12, wherein the DC voltage source is configured to selectively apply to a pair of electrodes, referred to as active electrodes, successive constant voltage steps, said electrodes of the pair constituting an anode and a cathode, wherein, for example, the voltage values of the successive steps are decreasing and/or last between 500 ms and 2 s each.

14. A measuring station according to any one of claims 1 to 13, further comprising a weight sensor.

15. A measuring station according to any one of claims 1, or 3 to 14, comprising:
- a base (102) comprising the left electrode group (LG) and the right electrode group (RG),
- a handle (110) with at least one electrode capable of coming into contact with the hand,
in which, in DC mode, the switch (566) is configured to connect all the electrodes of the base (102) to the terminals of the DC voltage source (806) and to connect the electrode of the handle (110) to a high impedance (HiZ) of more than 500 kOhm.
